# EUROPÄISCHE PATENTSCHRIFT

(11) **EP 1 809 236 B1**
(45) Veröffentlichungstag und Bekanntmachung des Hinweises auf die Patenterteilung: **26.12.2012**
(21) Anmeldenummer: 05747562.6
(22) Anmeldetag: 11.05.2005
(51) Int. Cl.: A61K 9/00

(54) **PULVERFORMULIERUNGEN FÜR DIE INHALATION, DIE ENANTIOMERENREINE BETAAGONISTEN ENTHALTEN**
INHALATION POWDER FORMULATIONS CONTAINING ENANTIOMERICALLY PURE BETA-AGONISTS
FORMULATIONS PULVERULENTES A INHALER CONTENANT DES BETA-AGONISTES ENANTIOMERIQUEMENT PURS

(30) Priorität: 14.05.2004 DE 102004024451
(43) Veröffentlichungstag der Anmeldung: 25.07.2007
(73) Patentinhaber: Boehringer Ingelheim International GmbH, 55216 Ingelheim (DE); Boehringer Ingelheim Pharma GmbH & Co. KG, 55216 Ingelheim (DE)
(72) Erfinder: TRUNK, Michael, 55218 Ingelheim (DE); SCHIEWE, Joerg, 55129 Mainz (DE)
(74) Vertreter: Simon, Elke Anna Maria
(86) Internationale Anmeldenummer: PCT/EP2005/005078
(87) Internationale Veröffentlichungsnummer: WO 2005/110359

(56) Entgegenhaltungen:
- EP-A- 0 073 505
- WO-A-2004/045618
- US-A- 4 460 581

## Beschreibung

Die vorliegende Erfindung betrifft Pulverformulierungen für die Inhalation, die enantiomerenreine Verbindungen der allgemeinen Formel **1** 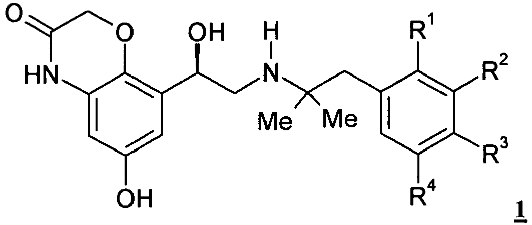 worin die Reste R¹, R², R³ und R⁴ die in den Ansprüchen und in der Beschreibung genannten Bedeutungen haben können, gegebenenfalls in Form Ihrer pharmazeutisch verträglichen Säureadditionssalze, sowie gegebenenfalls in Kombination mit einem pharmazeutisch verträglichen Hilfsstoff enthalten, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%, Verfahren zu deren Herstellung und deren Verwendung als Arzneimittel, insbesondere als Arzneimittel für die Behandlung von Atemwegserkrankungen

### Hintergrund der Erfindung

Betamimetika (ß-adrenerge Substanzen) sind aus dem Stand der Technik bekannt. Beispielsweise sei diesbezüglich auf die Offenbarung der US 4,460,581 sowie der EP 73505 verwiesen, die Betamimetika zur Therapie unterschiedlichster Erkrankungen vorschlagen.

Zur medikamentösen Therapie von Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.

Die vorliegende Erfindung zielt in einer besonders bevorzugten Ausführungsform auf Arzneimittelzubereitungen, die bei der Therapie von Atemwegserkrankungen einen therapeutischen Nutzen entfalten können.

Insbesondere bei der Behandlung von Atemwegserkrankungen bietet sich die inhalative Applikation des Wirkstoffs an. Neben der inhalativen Applikation von broncholytisch wirksamen Verbindungen in Form von Dosieraerosolen und Lösungen zur Inhalation kommt der Applikation von wirkstoffhaltigen Inhalationspulvern besondere Bedeutung zu.

Bei Wirkstoffen, die eine besonders hohe Wirksamkeit aufweisen, sind pro Einzeldosis zur Erzielung des therapeutisch erwünschten Effekts nur geringe Mengen des Wirkstoffs erforderlich. In solchen Fällen ist es notwendig, zur Herstellung des Inhalationspulvers den Wirkstoff mit geeigneten Hilfsstoffen zu verdünnen. Aufgrund des hohen Anteils an Hilfsstoff werden die Eigenschaften des Inhalationspulvers maßgeblich durch die Wahl des Hilfsstoffs beeinflußt. Bei der Wahl des Hilfsstoffs kommt dessen Korngröße eine besondere Bedeutung zu. Je feiner der Hilfsstoff desto schlechter sind in der Regel dessen Fließeigenschaften. Gute Fließeigenschaften sind allerdings Voraussetzung für eine hohe Dosiergenauigkeit bei der Abfüllung und Abteilung der einzelnen Präparatedosen, wie etwa bei der Herstellung von Kapseln (Inhaletten) zur Pulverinhalation oder der Dosierung eines Einzelhubes durch den Patienten vor der Anwendung eines Mehrdosisinhalators. Des weiteren ist die Korngröße des Hilfsstoffs von großer Bedeutung für das Entleerungsverhalten von Kapseln in einem Inhalator bei der Anwendung. Es hat sich ferner gezeigt, daß die Korngröße des Hilfsstoffs starken Einfluß auf den ausgebrachten inhalierbaren Wirkstoffanteil des Inhalationspulvers hat. Unter inhalierbarem bzw. inhalierfähigem Wirkstoffanteil werden die Teilchen des Inhalationspulvers verstanden, die beim Inhalieren mit der Atemluft tief in die Verästelungen der Lunge transportiert werden. Die hierzu erforderliche Teilchengröße liegt zwischen 0.5 und 10µm, vorzugsweise zwischen 1 und 6 µm.

Es ist Aufgabe der Erfindung, ein Betamimetikum-haltiges Inhalationspulver bereitzustellen, welches bei guter Dosiergenauigkeit (betreffend die pro Pulvercharge herstellerseitig oder durch den Patienten Inhalator-abhängig vor der Anwendung abgefüllte Menge an Wirkstoff und Pulvermischung wie auch die pro Applikation durch den Inhalationsvorgang ausgebrachte und lungengängige Wirkstoffmenge) und geringer chargenweisen Variabilität die Applikation des Wirkstoffs mit hohem inhalierfähigen Anteil erlaubt. Es ist ferner Aufgabe der vorliegenden Erfindung, ein Betamimetikum-haltiges Inhaltionspulver bereitzustellen, welches ein gutes Entleerungsverhalten der Kapseln gewährleistet, sollte es z.B. mittels eines Inhalators, wie er beispielsweise in der WO 94/28958 beschrieben wird, am Patienten oder in vitro über einen Impaktor oder Impinger zur Anwendung gelangen.

Daß Betamimetika gegebenenfalls bereits in sehr geringen Dosen eine hohe therapeutische Wirksamkeit aufweisen, stellt weitere Anforderungen an ein mit hoher Dosiergenauigkeit einzusetzendes Inhalationspulver. Aufgrund der geringen, zur Erzielung des therapeutischen Effekts erforderlichen Konzentration des Wirkstoffs im Inhalationspulver, muß ein hohes Maß an Homogenität der Pulvermischung und eine geringe Schwankung im Dispergierverhalten von Pulver- zu Pulvercharge gewährleistet werden. Die Homogenität der Pulvermischung wie auch gering schwankende Dispergiereigenschaften tragen entscheidend dazu bei, daß die Freisetzung des inhalierfähigen Anteils des Wirkstoffs reproduzierbar in gleichbleibend hohen Mengen und somit möglichst geringer Variabilität erfolgt.

Dementsprechend ist es ferner Aufgabe der vorliegenden Erfindung, ein Betamimetikum-haltiges Inhaltionspulver bereitzustellen, welches durch eine hohes Maß an Homogenität und Gleichförmigkeit der Dispergierbarkeit gekennzeichnet ist. Ferner zielt die vorliegende Erfindung auf die Bereitstellung eines Inhalationspulvers, welches die Applikation des inhalierfähigen Wirkstoffanteils bei möglichst geringer Variabilität erlaubt.

Wenn auch nicht ausschließlich, so aber doch insbesondere bei der Applikation von Inhalationspulvern mittels pulver-haltiger Kapseln, spielt das Entleerungsverhalten aus dem Pulverreservoir (das Behältnis, aus dem heraus das Wirkstoff-haltige Inhalationspulver zur inhalativen Applikation freigesetzt wird) eine bedeutsame Rolle. Wird die Pulverformulierung aus dem Pulverreservoir aufgrund eines nur geringen bzw. schlechten Entleerungsverhaltens nur in geringem Maße freigesetzt, bleiben bedeutsame Mengen des wirkstoffhaltigen Inhalationspulvers im Pulverreservoir (z.B. der Kapsel oder eines andersartigen Containers) zurück und können durch den Patienten nicht therapeutisch nutzbar gemacht werden. Dies hat zur Konsequenz, daß die Dosierung des Wirkstoffs in der Pulvermischung erhöht werden muß, damit die ausgebrachte Wirkstoffmenge zur Erzielung des therapeutisch gewünschten Effekts ausreichend hoch ist.

Vor diesem Hintergrund ist es eine weitere Aufgabe der vorliegenden Erfindung, eine Betamimetikum-haltiges Inhalationspulver bereitzustellen, welches ferner durch ein sehr gutes Entleerungsverhalten gekennzeichnet ist.

### Beschreibung der Erfindung

Die vorliegende Erfindung betrifft Inhalationspulver, enthaltend ein oder mehrere, bevorzugt eine enantiomerenreine Verbindung der allgemeinen Formel **1** 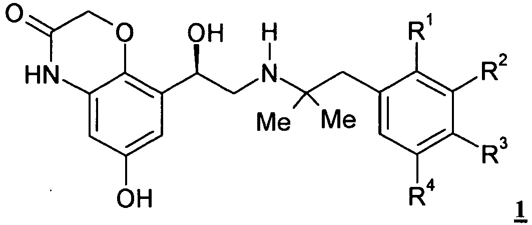 worin
- R¹: Wasserstoff, C₁-C₄-Alkyl, C₁-C₄-Alkoxy oder Halogen;
- R²: Wasserstoff, C₁-C₄-Alkyl, C₁-C₄-Alkoxy oder Halogen;
- R³: Wasserstoff, C₁-C₄-Alkyl, C₁-C₄-Alkoxy, Halogen, OH, -O-C₁-C₄-Alkylen-COOH oder -O-C₁-C₄-Alkylen-COO-C₁-C₄-alkyl;
- R⁴: Wasserstoff, C₁-C₄-Alkyl, C₁-C₄-Alkoxy oder Halogen,
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%.

Bevorzugt sind vorstehend genannte Inhalationspulver, die ein oder mehrere, bevorzugt eine enantiomerenreine Verbindung der allgemeinen Formel **1** enthalten, worin
- R¹: Wasserstoff oder Halogen;
- R²: Wasserstoff oder Halogen;
- R³: Wasserstoff, C₁-C₄-Alkoxy oder Halogen;
- R⁴: Wasserstoff oder Halogen;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%.

Bevorzugt sind Inhalationspulver, die ein oder mehrere, bevorzugt eine enantiomerenreine Verbindung der allgemeinen Formel **1** enthalten, worin
- R¹: Wasserstoff, Fluor oder Chlor, bevorzugt Wasserstoff oder Fluor;
- R²: Wasserstoff, Fluor oder Chlor, bevorzugt Wasserstoff oder Fluor;
- R³: Wasserstoff, Methoxy, Ethoxy, Fluor oder Chlor, bevorzugt Wasserstoff, Methoxy, Ethoxy oder Fluor;
- R⁴: Wasserstoff, Fluor oder Chlor, bevorzugt Wasserstoff oder Fluor;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%.

Bevorzugt sind Inhalationspulver, die ein oder mehrere, bevorzugt eine enantiomerenreine Verbindung der allgemeinen Formel **1** enthalten, worin
- R¹: Wasserstoff oder Fluor;
- R²: Wasserstoff;
- R³: Methoxy, Ethoxy oder Fluor;
- R⁴: Wasserstoff;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%.

Von erfindungsgemäß gleichrangiger Bedeutung sind ferner Inhalationspulver, die ein oder mehrere, bevorzugt eine enantiomerenreine Verbindung der allgemeinen Formel **1** enthalten, worin
- R¹: Wasserstoff;
- R²: Wasserstoff, Fluor oder Chlor, bevorzugt Wasserstoff oder Fluor;
- R³: Wasserstoff;
- R⁴: Wasserstoff, Fluor oder Chlor, bevorzugt Wasserstoff oder Fluor;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, dadurch gekennzeichnet, dass die Verbindungen der allgemeinen Formel **1** die Parameter aufweisen, charakteristische Partikelgröße X₅₀ von 0,1 µm bis 10µm und Q_{(5.8)} von größer 60%.

Erfindungsgemäß bevorzugt sind ferner Inhalationspulver, die ein oder mehrere, bevorzugt eine enantiomerenreine Verbindung der allgemeinen Formel **1** in Form ihrer freien Basen enthalten

Von erfindungsgemäß gleichrangiger Bedeutung sind ferner Inhalationspulver, die ein oder mehrere, bevorzugt eine enantiomerenreine Verbindung der allgemeinen Formel **1** in Form Ihrer pharmazeutisch verträglichen Säureadditionssalze enthalten, die durch die allgemeine Formel **1-HX** dargestellt werden können.

Bevorzugte Inhalationspulver enthalten als Saureadditionssalze eine oder mehrere, bevorzugt eine Verbindung der allgemeinen Formel **1-HX,** 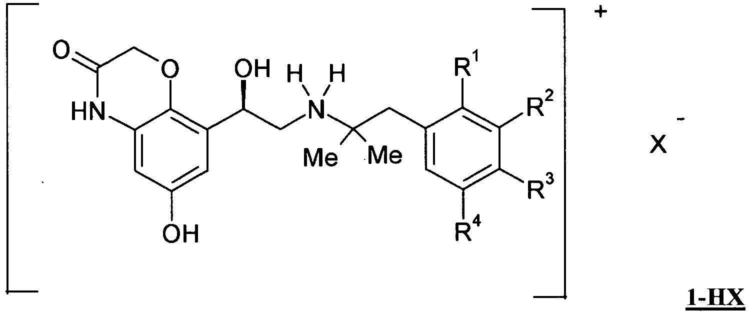 worin
- X⁻: ein einfach negativ geladenes Anion, bevorzugt ein einfach negativ geladenes Anion ausgewählt aus der Gruppe bestehend aus Chlorid, Bromid, Iodid, Sulfat, Phosphat, Methansulfonat, Nitrat, Maleinat, Acetat, Benzoat, Citrat, Salicylat, Trifluoracetat, Fumarat, Tartrat, Oxalat, Succinat, Benzoat und p-Toluolsulfonat,
bedeutet und die Reste R¹, R², R³ und R⁴ eine der vorstehend genannten Bedeutungen aufweisen können, gegebenenfalls in Form Ihrer Tautomere, Mischungen der Tautomere, Hydrate oder Solvate, sowie gegebenenfalls im Gemisch mit einem oder mehreren physiologisch unbedenklichen Hilfsstoffen.

Bevorzugte Inhalationspulver enthalten eine oder mehrere, bevorzugt eine Verbindung der Formel **1-HX,** worin
- X⁻: ein einfach negativ geladenes Anion ausgewählt aus der Gruppe bestehend aus Chlorid, Bromid, Sulfat, Methansulfonat, Maleinat, Acetat, Benzoat, Citrat, Salicylat, Trifluoracetat, Fumarat, Tartrat und Succinat;
bedeutet und die Reste R¹, R², R³ und R⁴ eine der vorstehend genannten Bedeutungen aufweisen können, gegebenenfalls in Form Ihrer Tautomere, Mischungen der Tautomere, Hydrate oder Solvate, sowie gegebenenfalls im Gemisch mit einem oder mehreren physiologisch unbedenklichen Hilfsstoffen.

Bevorzugte Inhalationspulver enthalten eine oder mehrere, bevorzugt eine Verbindung der Formel **1-HX**, worin
- X⁻: ein einfach negativ geladenes Anion ausgewählt aus der Gruppe bestehend aus Chlorid, Methansulfonat, Maleinat, Acetat, Citrat, Salicylat, Trifluoracetat, Fumarat und Succinat, bevorzugt Chlorid, Maleinat, Salicylat, Fumarat und Succinat, besonders bevorzugt Chlorid;
bedeutet und die Reste R¹, R², R³ und R⁴ eine der vorstehend genannten Bedeutungen aufweisen können, gegebenenfalls in Form Ihrer Tautomere, Mischungen der Tautomere, Hydrate oder Solvate, sowie gegebenenfalls im Gemisch mit einem oder mehreren physiologisch unbedenklichen Hilfsstoffen.

Besonders bevorzugt sind ferner Inhalationspulver, die ein oder mehrere, bevorzugt eine enantiomerenreine Verbindung der allgemeinen Formel **1** enthalten, die ausgewählt sind aus der Gruppe bestehend aus
- 6-Hydroxy-8-{(R)-1-hydroxy-2-[2-(4-methoxy-phenyl)-1,1-dimethyl-ethylamino]-ethyl}-4H-benzo[1,4]oxazin-3-on - Hydrochlorid;
- 8-{(R)-2-[2-(2,4-Difluor-phenyl)-1,1-dimethyl-ethylamino]-1-hydroxy-ethyl}-6-hydroxy-4H-benzo[1,4]oxazin-3-on-Hydrochlorid;
- 8-{(R)-2-[2-(3,5-Difluor-phenyl)-1,1-dimethyl-ethylamino]-1-hydroxy-ethyl}-6-hydroxy-4H-benzo[1,4]oxazin-3-on-Hydrochlorid;
- 8-{(R)-2-[2-(4-Ethoxy-phenyl)-1,1-dimethyl-ethylamino]-1-hydroxy-ethyl}-6-hydroxy-4H-benzo[1,4]oxazin-3-on-hydrochlorid;
- 8-{(R)-2-[2-(4-Fluor-phenyl)-1,1-dimethyl-ethylamino]-1-hydroxy-ethyl}-6-hydroxy-4H-benzo[1,4]oxazin-3-on- hydrochlorid;
- 6-Hydroxy-8-{(R)-1-hydroxy-2-[2-(4-methoxy-phenyl)-1,1-dimethyl-ethylamino]-ethyl}-4H-benzo[1,4,]oxazin-3-on maleinat;
- 6-Hydroxy-8-{(R)-1-hydroxy-2-[2-(4-methoxy-phenyl)-1,1-dimethyl-ethylamino]-ethyl}-4H-benzo[1,4,]oxazin-3-on salicylat;
- 6-Hydroxy-8-{(R)-1-hydroxy-2-[2-(4-methoxy-phenyl)-1,1-dimethyl-ethylamino]-ethyl}-4H-benzo[1,4,]oxazin-3-on Succinat;
- 6-Hydroxy-8-{(R)-1-hydroxy-2-[2-(4-methoxy-phenyl)-1,1-dimethyl-ethylamino]-ethyl}-4H-benzo[1,4,]oxazin-3-on-fumarat;
- 8-{(R)-2-[2-(2,4-Difluor-phenyl)-1,1-dimethyl-ethylamino]-1-hydroxy-ethyl}-6-hydroxy-4H-benzo[1,4]oxazin-3-on-maleinat;
- 8-{(R)-2-[2-(2,4-Difluor-phenyl)-1,1-dimethyl-ethylamino]-1-hydroxy-ethyl}-6-hydroxy-4H-benzo[1,4]oxazin-3-on-salicylat;
- 8-{(R)-2-[2-(2,4-Difluor-phenyl)-1,1-dimethyl-ethylamino]-1-hydroxy-ethyl}-6-hydroxy-4H-benzo[1,4]oxazin-3-on-succinat;
- 8-{(R)-2-[2-(2,4-Difluor-phenyl)-1,1-dimethyl-ethylamino]-1-hydroxy-ethyl}-6-hydroxy-4H-benzo[1,4]oxazin-3-on-fumarat;
- 8-{(R)-2-[2-(3,5-Difluor-phenyl)-1,1-dimethyl-ethylamino]-1-hydroxy-ethyl}-6-hydroxy-4H-benzo[1,4]oxazin-3-on-maleinat;
- 8-{(R)-2-[2-(3,5-Difluor-phenyl)-1,1-dimethyl-ethylamino]-1-hydroxy-ethyl}-6-hydroxy-4H-benzo[1,4]oxazin-3-on-salicylat;
- 8-{(R)-2-[2-(3,5-Difluor-phenyl)-1,1-dimethyl-ethylamino]-1-hydroxy-ethyl}-6-hydroxy-4H-benzo[1,4]oxazin-3-on-succinat;
- 8-{(R)-2-[2-(3,5-Difluor-phenyl)-1,1-dimethyl-ethylamino]-1-hydroxy-ethyl}-6-hydroxy-4H-benzo[1,4]oxazin-3-on-fumarat;
- 8-{(R)-2-[2-(4-Ethoxy-phenyl)-1,1-dimethyl-ethylamino]-1-hydroxy-ethyl}-6-hydroxy-4H-benzo[1,4]oxazin-3-on-maleinat;
- 8-{(R)-2-[2-(4-Ethoxy-phenyl)-1,1-dimethyl-ethylamino]-1-hydroxy-ethyl}-6-hydroxy-4H-benzo[1,4]oxazin-3-on-salicylat;
- 8-{(R)-2-[2-(4-Ethoxy-phenyl)-1,1-dimethyl-ethylamino]-1-hydroxy-ethyl}-6-hydroxy-4H-benzo[1,4]oxazin-3-on-succinat;
- 8-{(R)-2-[2-(4-Ethoxy-phenyl)-1,1-dimethyl-ethylamino]-1-hydroxy-ethyl}-6-hydroxy-4H-benzo[1,4]oxazin-3-on-fumarat;
- 8-{(R)-2-[2-(4-Fluor-phenyl)-1,1-dimethyl-ethylamino]-1-hydroxy-ethyl}-6-hydroxy-4H-benzo[1,4]oxazin-3-on-maleinat;
- 8-{(R)-2-[2-(4-Fluor-phenyl)-1,1-dimethyl-ethylamino]-1-hydroxy-ethyl}-6-hydroxy-4H-benzo[1,4]oxazin-3-on-salicylat;
- 8-{(R)-2-[2-(4-Fluor-phenyl)-1,1-dimethyl-ethylamino]-1-hydroxy-ethyl}-6-hydroxy-4H-benzo[1,4]oxazin-3-on-succinat und
- 8-{(R)-2-[2-(4-Fluor-phenyl)-1,1-dimethyl-ethylamino]-1-hydroxy-ethyl}-6-hydroxy-4H-benzo[1,4]oxazin-3-on-fumarat,
gegebenenfalls in Form Ihrer Tautomere, Mischungen der Tautomere, Hydrate oder Solvate.

In den erfindungsgemäßen Inhalationspulvern sind die enantiomerenreinen Verbindungen der allgemeinen Formel **1**, worin R¹, R², R³ R⁴ die vorstehend genannten Bedeutungen haben, in kristalliner Form, gegebenenfalls in Form ihrer kristallinen Tautomere, kristallinen Hydrate oder kristallinen Solvate enthalten. Besonders bevorzugt sind enantiomerenreine, kristalline Verbindungen der allgemeinen Formel **1** bei denen R¹, R², R³ und R⁴ die vorstehend genannten Bedeutungen haben, gegebenenfalls in Form Ihrer kristallinen Tautomere, kristallinen Hydrate oder kristallinen Solvate, enthalten, die ferner dadurch gekennzeichnet sind, dass es sich um kristalline Verbindungen handelt, die lediglich in einer einzigen Kristallmodifikationen vorliegen.

Unter der Bezeichnung eine einzige Kristallmodifikation werden dabei kristalline Verbindungen der Formel **1** verstanden, die nicht ein Gemisch gegebenenfalls existierender polymorpher Kristallmodifikationen und / oder Gemische aus einer oder mehreren Kristallmodifikationen mit dem amorphen bzw. glassartigem Zustand der Verbindungen gemäß Formel **1** darstellen.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, n-Propylen oder n-Butylen.

Als Alkyloxygruppen (oder auch -O-Alkylgruppen) werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Halogen steht im Rahmen der vorliegenden Erfindung für Fluor, Chlor, Brom oder Jod. Sofern nicht gegenteilig angegeben, gelten Fluor, Chlor und Brom als bevorzugte Halogene.

Die Bezeichnung enantiomerenrein beschreibt im Rahmen der vorliegenden Erfindung Verbindungen der Formel **1**, die in einer Enantiomerenreinheit von wenigstens 85%ee, bevorzugt von wenigstens 90%ee, besonders bevorzugt von ≥ 95%ee vorliegen. Die Bezeichnung ee (enantiomeric excess) ist im Stand der Technik bekannt und beschreibt den optischen Reinheitsgrad chiraler Verbindungen.

Die hochkristallinen Verbindungen der Formel **1** sind wie nachfolgend dargestellt erhältlich (Schema 1).

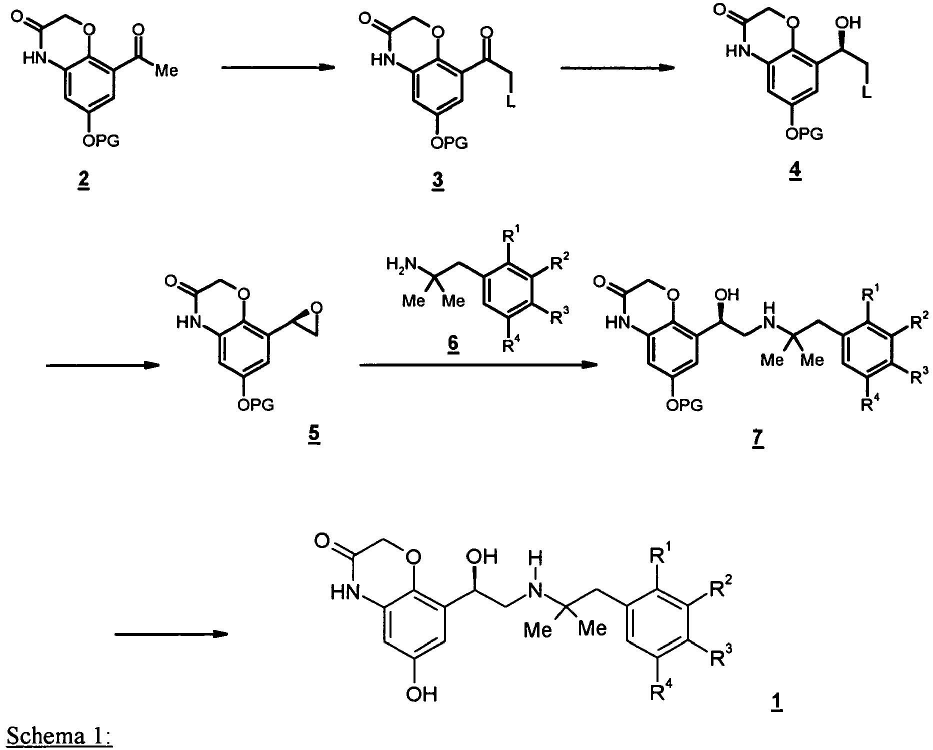

In den in Schema 1 genannten Verbindungen der Formeln **2** bis **5** und **7** steht die Gruppe OPG für eine durch eine Schutzgruppe (PG) geschützte Hydroxylfunktion. Zur Auswahl geeigneter Schutzgruppen für die Hydroxylgruppe sei dabei auf den Stand der Technik verwiesen, wie er beispielsweise in Protective Groups in Organic Synthesis, T.W. Greene und P.G.M. Wuts, John Wiley & Sons Inc, third Edition, 1999 dargelegt ist.

Bevorzugt steht OPG für eine Gruppe, die ausgewählt ist aus der Gruppe bestehend aus -O-C₁-C₄-Alkyl, -O-Benzyl oder -O-CO-C₁-C₄-Alkyl, bevorzugt -O-Methyl, -, -O-Benzyl oder -O-Acetyl, besonders bevorzugt -O-Methyl oder -O-Benzyl, besonders bevorzugt -O-Benzyl.

In den in Schema 1 genannten Verbindungen der Formeln **3** und **4** steht die Gruppe L für eine Abgangsgruppe. Bevorzugt steht L für eine Abgangsgruppe, die ausgewählt ist aus der Gruppe bestehend aus
Chlor, Brom, Iod, Methansulfonat, Trifluormethansulfonat und p-Toluolsulfonat, bevorzugt Chlor oder Brom, besonders bevorzugt Chlor.

In den in Schema 1 genannten Verbindungen der Formeln **6** und **7** können die Reste R¹, R², R³ und R⁴ die vorstehend genannten Bedeutungen aufweisen.

Ausgehend von 8-Acetyl-6-benzyloxy-4H-benzo[1,4]oxazin-3-on (**2**) erfolgt die Darstellung der Verbindungen der allgemeinen Formel **3** in aus dem Stand der Technik bekannter Art und Weise. Die Verbindung der Formel **3** wird dann in Gegenwart eines chiralen Übergangsmetallkatalysators enantioselektiv in den chiralen Alkohol der allgemeinen Formel **4** überführt, der dann unter geeigneten Bedingungen zum chiralen Oxiran der Formel **5** reagiert. Verfahren zur Durchführung der Synthese von Oxiranen ausgehend von Derivaten der Verbindung der Formel **3** sind im Stand der Technik bekannt (siehe beispielsweise Hamada et al., Org. Letters 2002, 4, 4373-4376).

Durch Umsetzung der Oxirane **5** mit den Aminen der Formel **6** werden die Verbindungen der Formel **7** erhalten, die nach Abspaltung der Schutzgruppe (PG) in die Verbindungen der Formel **1** überführt werden können. Sofern die Verbindungen der Formel **1** nach dem oben skizzierten Syntheseverfahren gegebenenfalls nicht in kristalliner Form erhalten werden, kann sich an die Synthese eine Umkristallisation aus geeigneten Lösemitteln anschließen. Detalliertere Ausführungen hierzu sind dem nachstehenden experimentellen Teil der vorliegenden Erfindung zu entnehmen.

Zur Herstellung der erfindungsgemäßen Inhalationspulver, ist es zunächst erforderlich, die in kristalliner Form erhaltenen Verbindungen der Formel **1** in feinteiliger (bzw. mikronisierter) Form bereitzustellen.

Zur Durchführung des Mikronisierungs- bzw. Mahlprozesses können gängige Mühlen zum Einsatz gelangen. Bevorzugt wird die Mikronisierung dabei unter Feuchtigkeitsausschluß durchgeführt, besonders bevorzugt unter Einsatz eines entsprechenden Inertgases, wie beispielsweise Stickstoff. Als besonders bevorzugt hat sich die Verwendung von Luftstrahlmühlen erwiesen, in denen die Zerkleinerung des Mahlguts durch Aufeinenderprallen der Partikel miteinender sowie Aufprall der Partikel auf die Wände des Mahlbehälters erfolgt. Die Durchführung des Mikronisierens ist dabei sowohl mittels sogenannter Gegenstrahlmühlen, gegebenfalls mit nachfolgendem Sichten als auch in bevorzugter Art und Weise mittels Spiralluftstrahlmühlen möglich. Als Mahlgas gelangt erfindungsgemäß bevorzugt Stickstoff zur Anwendung. Das Mahlgut wird mittels des Mahlgases unter spezifischen Drücken (Mahldruck) gefördert. Im Rahmen der vorliegenden Erfindung wird der Mahldruck üblicherweise auf einen Wert zwischen etwa 2 und etwa 8 bar, bevorzugt zwischen etwa 3 und etwa 7 bar, besonders bevorzugt zwischen etwa 3,5 und etwa 6,5 bar eingestellt. Der Eintrag des Mahlgutes in die Luftstrahlmühle erfolgt mittels des Speisegases unter spezifischen Drücken (Speisedruck). Im Rahmen der vorliegenden Erfindung hat sich eine Speisedruck zwischen etwa 2 und etwa 8 bar, bevorzugt zwischen etwa 3 und etwa 7 bar, besonders bevorzugt zwischen etwa 3,5 und etwa 6 bar bewährt. Als Speisegas gelangt vorzugsweise ebenfalls ein Inertgas, besonders bevorzugt ebenfalls Stickstoff zur Anwendung. Die Zufuhr des Mahlguts (kristalline Verbindungen gemäß Formel **1**) kann dabei in einer Förderräte von etwa 5 - 45 g/min, vorzugsweise mit etwa 15-35 g/min erfolgen.

Beispielsweise und ohne den Gegenstand der Erfindung darauf zu beschränken, hat sich als eine mögliche Ausführungsform einer Luftstrahlmühle das folgende Gerät bewährt: 2-Zoll Microniser mit Mahlring 0,8 mm-Bohrung, Firma Sturtevant Inc., 348 Circuit Street, Hanover, MA 02239, USA. Unter Verwendung dieses Geräts wird der Mahlprozess vorzugsweise mit folgenden Mahlparametern durchgeführt:
Mahldruck: etwa 4,5 - 6,5 bar; Speisedruck: etwa 4,5 - 6,5 bar: Zufuhr des Mahlguts: etwa 17 - 21 g/min.

Als weiteres Beispiel kann die Verwendung einerLuftstrahlmühle der Firma Jetpharma, Typ Jetmill MC 50 genannt werden, die mit folgenden Prozeßparametern betrieben werden kann:

| | |
|---|---|
| Mahldruck: | 8,0 bar (+/- 0,5 bar) |
| Speisedruck: | 8,5 bar (+/- 0,5 bar), |
| | Anmerkung: der Speisedruck wird immer um 0,25 bis 0,5 bar höher als der Mahldruck eingestellt |
| Produktzufuhr: | 20 g/min (+/- 2,0 g/min) |
| Düseneinstellung (Injektor): | 37,2 mm (konstant) |

Das so erhaltene Mahlgut wird anschließend unter den nachfolgend genannten spezifischen Bedingungen weiterverarbeitet. Hierzu wird das Mikronisat bei einer Temperatur von 15 - 50 °C, vorzugsweise 20 - 45 °C, besonders bevorzugt bei 25 - 40 °C Wasserdampf einer relativen Feuchte von wenigstens 40% ausgesetzt. Vorzugsweise wird die Feuchte auf einen Wert von 50 - 95% r.F., bevorzugt auf 60 - 90 % r.F., besonders bevorzugt auf 70 - 85 % r.F. eingestellt.

Unter relativer Feuchte (r.F.) wird im Rahmen der vorliegenden Erfindung der Quotient des Partialdruckes des Wasserdampfs und des Dampfdruckes des Wassers bei der betreffenden Temperatur verstanden. Vorzugsweise wird das aus vorstehend beschriebenem Mahlprozess erhältliche Mikronisat den oben genannten Raumbedingungen wenigstens über einen Zeitraum von 6 Stunden ausgesetzt. Bevorzugt wird das Mikronisat den genannten Raumbedingungen allerdings für etwa 12 bis etwa 120 Stunden, vorzugsweise etwa 15 bis etwa 96 Stunden, besonders bevorzugt etwa 18 bis etwa 72 Stunden ausgesetzt. In einer Variante wird diesem Verfahrensschritt eine Nachtrocknung angeschlossen. Das Mahlgut wird dabei einer erhöhten Temperatur ausgesetzt. Hierzu wird über eine Zeitdauer von midestens 0.5 Stunden, bevorzugt von 0.5 Stunden bis 6 Stunden, besonders bevorzugt von 0.5 Stunden bis 3 Stunden das Mikronisat einer erhöhten Temperatur von mindestens 40°C, bevorzugt mindestens 50°C und maximal 70°C bei reduzierter relativer Feuchtigkeit, das heißt einer relativen Feuchtigkeit kleiner 60%, bevorzugt kleiner 40% und besonders bevorzugt kleiner 30% ausgesetzt.

Die gemäß vorstehender Vorgehensweise erhältlichen erfindungsgemäßen Mikronisate der Verbindungen der Formel **1** weisen eine charakteristische Partikelgröße X₅₀ von zwischen zwischen 0,5µm und 6 µm, besonders bevorzugt zwischen 1,0 µm und 3,5µm auf. Darüberhinaus sind sie gekennzeichnet durch den Parameter Q_{(5.8)} von größer 70 %, besonders bevorzugt größer 80%.

Dabei bezeichnet der Kennwert X₅₀ den Medianwert der Teilchengröße, unterhalb derer 50% der Teilchenmenge bezüglich der Volumenverteilung der einzelnen Teilchen liegt. Der Kennwert Q_{(5.8)} entspricht der Teilchenmenge der Partikel, die bezogen auf die Volumenverteilung der Partikel unterhalb von 5.8 µm liegt. Die Partikelgrößen wurden im Rahmen der vorliegenden Erfindung mittels Laserbeugung (Fraunhoferbeugung) bestimmt. Zur Bestimmung der Partikelgrössen mittels Laserbeugung (Fraunhoferbeugung) wurde unter Anwendung der in der WO 03/078429 (Seite 16 ff) beschriebenen Vorgehensweise verfahren.

Die vorstehend beschriebenen Mikronisate der Verbindungen der allgemeinen Formel **1** können gegebenenfalls ohne weiteren Hilfsstoff zur inhalativen Applikation gelangen. Vorzugsweise enthalten die erfindungsgemäßen Arzneimittel allerdings neben einer oder mehreren, bevorzugt einer Verbindung der Formel **1** wenigstens einen physiologisch unbedenklichen Hilfsstoff oder ein Gemisch physiologisch unbedenklicher Hilfsstoffe.

Als physiologisch unbedenkliche Hilfsstoffe, die zur Darstellung der erfindungsgemäßen Inhalationspulver zur Anwendung gelangen können, seien beispielsweise genannt:
Monosaccharide (z.B. Glucose, Fructose oder Arabinose), Disaccharide (z.B. Lactose, Saccharose, Maltose oder Trehalose), Oligo- und Polysaccharide (z.B. Maltodextrin, Stärke, Cellulose und deren Derivate), Polylaktid-/glycolid (Resomer), Polyalkohole (z.B. Sorbitol, Mannitol, Xylit), Aminosäuren (Argininhydrochlorid), Chitosan (besonders bevorzugt Laktose, Mannitol, Saccharose, Sorbit, Trehalose), Alkali- und Erdalkalisalzen der Stearinsäure (z.B. MgStearat), Salze (z.B. Natriumchlorid, Calciumcarbonat) oder Mischungen dieser Hilfsstoffe miteinander. Bevorzugt gelangen Mono-, Disaccharide oder Polyalkohole zur Anwendung, wobei die Verwendung von Lactose, Glucose, Trehalose oder Mannitol, bevorzugt Lactose, Mannitol oder Glucose, insbesondere, aber nicht ausschließlich in Form ihrer Hydrate, bevorzugt ist. Als besonders bevorzugt im Sinne der Erfindung gelangen Lactose oder Mannitol, höchst bevorzugt Lactosemonohydrat oder Mannitol als Hilfsstoff zur Anwendung.

In solchen erfindungsgemäßen Arzneimittelformulierungen, die neben einer Verbindung der Formel **1** ferner einen physiologisch verträglichen Hilfsstoff enthalten, liegt das Verhältnis von Verbindung der Formel **1** zu Hilfsstoff üblicherweise in einem Bereich von 5:100 bis 1:100000, bevorzugt 3:1000 bis 1:10000 und besonders bevorzugt von 1:1000 bis 3:10000 gemischt wird, wobei die oben genannten Verhältnisse Massenverhältnisse (w/w) darstellen.

Die erfindungsgemäßen Inhalationspulver werden pro inhalativer Applikation üblicherweise in Mengen von 3-100 mg, bevorzugt 5-50 mg appliziert.

Enthalten die erfindungsgemäßen Inhalationspulver keinen Hilfsstoff sondern lediglich, ein oder mehrere, bevorzugt eine Verbindung der Formel **1** in mikronisierter Form, so werden pro inhalativer Applikation üblicherweise 1 - 30µg, bevorzugt 3 - 25µg und besonders bevorzugt 5 - 20µg Inhalationspulver appliziert.

Die erfindungsgemäßen Inhalationspulver werden in bevorzugter Art und Weise in Form einer pre-metered Arzneiform zur Verfügung gestellt. Als Beispiel kann hierfür ein Inhalationskapselsystem genannt werden. Ebenso sind Systeme denkbar, bei denen die Pulverzubereitung z.B. in Form der Befüllung von Blisternäpfen in Einzeldosen bereitgestellt wird. In einer weiteren Form eignen sich die erfindungsgemäßen Zubereitungen auch für die Anwendung derselben in Inhalatoren, die ein Pulverreservoir aufweisen und erst direkt vor der Anwendung die zu applizierende Pulvermenge beziehungsweise das kristalline Mikronisat des Wirkstoffes dosiert / abgeteilt wird. Die hier beschriebenen Pulverzubereitungen können mittels eines geeigneten Inhalators inhaliert werden. Geeignete Inhalatoren sind aus dem Stand der Technik bekannt. Besonders geeignete Inhalatoren sind beispielsweise in der WO 03/084502 genannt, auf die hinsichtlich der dort offenbarten Inhalatoren an dieser Stelle vollinhaltlich Bezug genommen wird.

Die Darstellung der erfindungsgemäßen Inhalationspulver kann wie nachfolgend beschrieben erfolgen.

Das Verfahren zur Herstellung der erfindungsgemäßen Inhalationspulver ist dadurch gekennzeichnet, daß N+m etwa gleichgroße Portionen des physiologisch verträglichen Hilfsstoffs und N gleichgroße Portionen der mikroniserten Verbindung der Formel **1** abwechselnd schichtweise in ein geeignetes Mischgefäß gegeben werden und nach vollständiger Zugabe die 2N+m Schichten der beiden Komponenten mittels eines geeigneten Mischers gemischt werden, wobei zunächst die Zugabe einer Portion des physiologisch verträglichen Hilfsstoffs erfolgt, wobei N eine ganze Zahl >0, vorzugsweise >1 ist und wobei m 0 oder 1 bedeutet.

Bevorzugt erfolgt die schichtweise Zugabe der einzelnen Fraktionen über eine geeignete Siebeinrichtung. Gegebenenfalls kann die gesamte Pulvermischung nach beendetem Mischvorgang noch einem oder mehreren weiteren Siebvorgängen unterworfen werden. Im erfindungsgemäßen Verfahren ist N naturgemäß unter anderem von der Gesamtmenge der herzustellenden Pulvermischung abhängig. Bei der Herstellung kleinerer Chargen kann bereits mit kleinerem N der gewünschte Effekt einer hohen Homogenität im Sinne der Gehaltsgleichförmigkeit erzielt werden. Prinzipiell ist es aber insbesondere bei größeren Chargen erfindungsgemäß bevorzugt, wenn N wenigstens 10 oder mehr, besonders bevorzugt 20 oder mehr, ferner bevorzugt 30 oder mehr beträgt. Je größer N, und damit verbunden, je größer die Gesamtzahl der gebildeten Schichten der Pulverfraktionen ist, umso homogener im Sinne der Gehaltsgleichförmigkeit wird die Pulvermischung.

Die Zahl m kann im Rahmen des erfindungsgemäßen Verfahren 0 oder 1 bedeuten. Steht m für 0 ist die letzte Fraktion, die schichtweise in die geeignete Mischapparatur zugegeben, vorzugsweise eingesiebt wird, die letzte Portion der mikronisierten Verbindung der Formel **1**. Steht m für die Zahl 1, wird als letzte Fraktion, die schichtweise in die geeignete Mischapparatur zugegeben, vorzugsweise eingesiebt wird, die letzte Portion des physiologisch verträglichen Hilfsstoffs zugegeben. Dies kann sich insofern als vorteilhaft erweisen, als im Falle von m = 1 mittels der letzten Portion Hilfsstoff eventuell noch in der Siebeinheit befindliche Reste der letzten Fraktion des Wirkstoffs mit in die Mischeinheit befördert werden können.

Vorzugsweise wird die erste Portion der N+m Portionen des Hilfsstoffs vorgelegt und anschließend die erste Portion der N Portionen des Wirkstoffs in den Mischbehälter eingebracht. Während im Rahmen des erfindungsgemäßen Verfahrens die Zugabe der einzelnen Komponenten üblicherweise in etwa gleichgroßen Portionen erfolgt, kann es gegebenenfalls vorteilhaft sein, wenn die erste der N+m Portionen Hilfsstoff, die in die Mischapparatur vorgelegt wird, ein größeres Volumen aufweist als die folgenden Portionen Hilfsstoff.

Die erfindungsgemäßen Inhalationspulver können ferner dadurch hergestellt werden, dass zunächst eine Wirkstoff-Hilfsstoffmischung gemäß der vorstehend erläuterten Vorgehensweise dargestellt wird und die so erhaltene Mischung anschließend erneut mit weiterem Hilfsstoff gemischt wird. Hierbei kann dann erneut die vorstehend genannte Vorgehensweise zur Anwendung gelangen, wobei dann N Portionen der Wirkstoff-Hilfsstoffmischung mit N+m Portionen weiteren Hilfsstoffs schichtweise gemischt werden.

Der in die erfindungsgemäßen Inhalationspulver eingesetzte Hilfsstoff weist vorzugsweise eine mittlere Teilchengröße von etwa 17 -120 µm, bevorzugt von etwa 17- 90 µm, besonders bevorzugt von etwa 20-60µm auf. Gegebenenfalls kann der Hilfsstoff auch aus einem Gemisch von gröberem Hilfsstoff mit einer mittleren Teilchengröße von 17 bis 75µm und feinerem Hilfststoff mit einer mittleren Teilchengröße von 1 bis 9 µm bestehen, wobei der Anteil von feinerem Hilfsstoff an der Gesamthilfsstoffmenge 1 bis 20 % betragen kann. Enthalten die mittels des erfindungsgemäßen Verfahrens herstellbaren Inhalationspulver ein Gemisch aus gröberen und feineren Hilfsstofffraktionen, so ist erfindungsgemäß die Herstellung solcher Inhalationspulver bevorzugt, in denen der gröbere Hilfsstoff eine mittlere Teilchengröße von 17 bis 50 µm, besonders bevorzugt von 20 bis 30 µm und der feinere Hilfststoff eine mittlere Teilchengröße von 2 bis 8 µm, besonders bevorzugt von 3 bis 7 µm aufweist. Dabei wird unter der mittleren Teilchengröße im hier verwendeten Sinne der 50 %-Wert aus der Volumenverteilung gemessen mit einem Laserdiffraktometer nach der Trockendispersionsmethode verstanden. Zur Bestimmung der mittleren Teilchengrösse nach dieser Methodik sei wiederum auf die Offenbarung der WO 03/078429 (Seite 21 ff) verwiesen.

Im Fall einer Hilfsstoffmischung aus gröberen und feineren Hilfsstoffanteilen, sind erfindungsgemäß solche Verfahren bevorzugt, mit denen Inhalationspulver erhalten werden, bei denen der Anteil von feinerem Hilfsstoff an der Gesamthilfsstoffmenge 3 bis 15 %, besonders bevorzugt 5 bis 10 % beträgt.

Bei den im Rahmen der vorliegenden Erfindung genannten prozentualen Angaben, handelt es sich stets um Gewichtsprozent.

Soll als Hilfsstoff eines der vorstehend genannten Gemische von gröberem Hilfsstoff mit feinerem Hilfsstoff zum Einsatz gelangen, bietet sich erfindungsgemäß an, die Hilfsstoffmischung ebenfalls mittels des erfindungsgemäßen Verfahrens aus N etwa gleichgroßen Portionen der feineren Hilfsstofffraktion mit N+m etwa gleichgroßen Portionen der gröberen Hilfsstofffraktion herzustellen. In einem solchen Fall ist es ratsam, zunächst die vorstehend genannte Hilfsstoffmischung aus den genannten Hilfsstofffraktionen zu generieren, und aus dieser anschließend mittels des erfindungsgemäßen Verfahrens die Gesamtmischung mit dem Wirkstoff zu erzeugen. Beispielsweise kann die Hilfsstoffmischung unter Anwendung des erfindungsgemäßen Verfahrens wie folgt erhalten werden. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm. Vorzugsweise wird die erste Fraktion der N+m Portionen des gröberen Hilfsstoffs vorgelegt und anschließend die erste Portion der N Portionen des feineren Hilfsstoffanteils in den Mischbehälter eingebracht. Die Zugabe der beiden Komponenten erfolgt abwechselnd durch schichtweises Einsieben der beiden Komponenten.

Nach Herstellung der Hilfsstoffmischung erfolgt aus dieser und dem gewünschten Wirkstoff die Herstellung des Inhaltionspulvers unter Anwendung des erfindungsgemäßen Verfahrens. Die Zugabe der beiden Komponenten erfolgt vorzugsweise über einen Siebgranulator mit einer Maschenweite von 0,1 bis 2 mm, besonders bevorzugt 0,3 bis 1 mm, höchst bevorzugt 0,3 bis 0,6 mm.

Vorzugsweise wird die erste Portion der N+m Portionen der Hilfsstoffmischung vorgelegt und anschließend die erste Portion der N Portionen des Wirkstoffs in den Mischbehälter eingebracht. Vorzugsweise erfolgt die Zugabe der beiden Komponenten über eine Siebeinheit abwechselnd und schichtweise in mehr als 20, vorzugsweise mehr als 25, besonders bevorzugt mehr als 30 Schichten. Beispielsweise kann bei einer gewünschten Gesamtpulvermenge von 30-35 kg, die beispielsweise 0,3-0,5% Wirkstoff enthält, sowie unter Verwendung gängiger Hilfsstoffe ein Einsieben der beiden Komponenten in je etwa 30 bis 60 Schichten (N = 30-60) erfolgen. Eine Durchführung des Verfahrens mit N>60 ist, wie für den Fachmann klar ersichtlich, ebenfalls möglich, um den gewünschten Effekt einer möglichst hohen Homogenität der Pulvermischung zu erzielen.

Die erfindungsgemäßen Inhalationspulver zeichnen sich durch vielfältige Anwendungsmöglichkeiten auf therapeutischem Gebiet aus. Hervorzuheben sind erfindungsgemäß solche Anwendungsmöglichkeiten, für welche die erfindungsgemäßen Verbindungen der Formel **1** aufgrund ihrer pharmazeutischen Wirksamkeit als Betamimetikum bevorzugt zur Anwendung gelangen können.

Ein weiterer Aspekt der vorliegenden Erfindung betrifft dementsprechend die vorstehend genannten Inhalationspulver als Arzneimittel. Die vorliegende Erfindung betrifft ferner die Verwendung der vorstehend genannten Inhalationspulver zur Herstellung eines Arzneimittels zur Behandlung von Atemwegserkrankungen.

Die vorliegende Erfindung betrifft bevorzugt die Verwendung der vorstehend genannten Inhalationspulver zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt sind die erfindungsgemäßen Inhalationspulver zur Verwendung zur Behandlung von Obstruktive Lungenerkrankungen die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt sind die erfindungsgemäßen Inhalationspulver zur Verwendung zur Behandlung von Lungenemphysemen die ihren Ursprung haben in COPD (chronisch obstruktive pulmonale Erkrankung) oder α1-Proteinase-Inhibitor-Mangel.

Bevorzugt sind die erfindungsgemäßen Inhalationspulver zur Verwendung zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt sind die erfindungsgemäßen Inhalationspulver zur Verwendung zur Behandlung von Interstitiellen Lungenerkrankungen, die ausgewählt sind aus der Gruppe bestehend aus infektiös bedingte Pneumonien, wie beispislweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt sind die erfindungsgemäßen Inhalationspulver zur Verwendung zur Behandlung von Zystischer Fibrose bzw. Mukoviszidose.

Bevorzugt sind die erfindungsgemäßen Inhalationspulver zur Verwendung zur Behandlung von Bronchitiden, wie beispielsweise

Bronchitis aufgrund bakterieller oder viraler Infektion, Allergische Bronchitis und Toxische Bronchitis.

Bevorzugt sind die erfindungsgemäßen Inhalationspulver zur Verwendung zur Behandlung von Bronchiektasen.

Bevorzugt sind die erfindungsgemäßen Inhalationspulver zur Verwendung zur Behandlung von ARDS (adult respiratory distress syndrom).

Bevorzugt sind die erfindungsgemäßen Inhalationspulver zur Verwendung zur Behandlung von Lungenödemen, beispielsweise
toxischer Lungenödeme nach Aspiration oder Inhalation von toxischen Substanzen und Fremdstoffen.

Bevorzugt sind die erfindungsgemäßen Inhalationspulver zur Verwendung zur Behandlung von Asthma oder COPD. Von besonderer Bedeutung ist ferner die vorstehend genannte Verwendung der erfindungsgemäßen Inhalationspulver zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Inhalationspulver zur Verwendung zur Behandlung der vorstehend genannten Erkrankungen, dadurch gekennzeichnet, daß eine oder mehrere der vorstehend genannten erfindungsgemäßen Inhalationspulver in therapeutisch wirksamen Mengen appliziert werden. Die vorliegende Erfindung betrifft bevorzugt ein Inhalationspulver zur Verwendung zur Behandlung von Asthma oder COPD, dadurch gekennzeichnet, daß eine oder mehrere der vorstehend genannten erfindungsgemäßen Inhalationspulver in therapeutisch wirksamen Mengen einmal täglich appliziert werden.

Die nachstehend beschriebenen Synthesebeispiele dienen der weitergehenden Illustration der vorliegenden Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Darstellung der Verbindungen der allgemeinen Formel 1:

### Beispiel 1: 6-Hydroxy-8-{(R)-1-hydroxy-2-[2-(4-methoxy-phenyl)-1,1-dimethyl-ethylamino]-ethyl}-4H-benzo[1,4]oxazin-3-on - Hydrochlorid

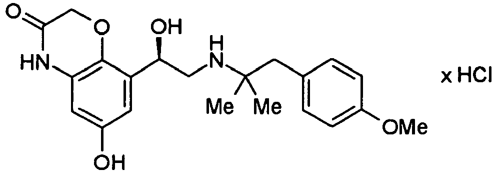

### a) 1-(5-Benzyloxy-2-hydroxy-3-nitro-phenyl)-ethanon

Zu einer Lösung von 81.5 g (0.34 mol) 1-(5-Benzyloxy-2-hydroxy-phenyl)-ethanon in 700 mL Essigsäure werden unter Kühlung mit dem Eisbad 18 mL rauchende Salpetersäure so zugetropft, dass die Temperatur nicht über 20°C steigt. Anschließend wird die Reaktionsmischung zwei Stunden bei Raumtemperatur gerührt, auf Eiswasser gegossen und filtriert. Das Produkt wird in Isopropanol umkristallisiert, abgesaugt und mit Isopropanol und Diisopropylether gewaschen. Ausbeute: 69.6 g (72%); Massenspektroskopie [M+H]⁺ = 288.

### b) 1-(3-Amino-5-benzyloxy-2-hydroxy-phenyl)-ethanon

69.5 g (242 mmol) 1-(5-Benzyloxy-2-hydroxy-3-nitro-phenyl)-ethanon werden in 1.4 L Methanol gelöst und in Gegenwart von 14 g Rhodium auf Kohle (10%ig) als Katalysator bei 3 bar und Raumtemperatur hydriert. Anschließend wird der Katalysator abfiltriert und das Filtrat eingeengt. Der Rückstand wird ohne zusätzliche Aufreinigung weiter umgesetzt. Ausbeute: 60.0 g (96%), R_{f}-Wert = 0.45 (Dichlormethan auf Kieselgel).

### c) 8-Acetyl-6-benzyloxy-4H-benzo[1,4]oxazin-3-on

Zu 60.0 g (233 mmol) 1-(3-Amino-5-benzyloxy-2-hydroxy-phenyl)-ethanon und 70.0 g (506 mmol) Kaliumcarbonat werden unter Kühlung mit dem Eisbad 21.0 mL (258 mmol) Chloracetylchlorid getropft. Anschließend wird über Nacht bei Raumtemperatur und dann 6 Stunden unter Rückfluß gerührt. Die heiße Reaktionsmischung wird filtriert, dann auf ca. 400 mL eingeengt und mit Eiswasser versetzt. Der ausfallende Niederschlag wird abgesaugt, getrocknet und durch Chromatographie an einer kurzen Kieselgelsäule (Dichlormethan:Methanol = 99:1) gereinigt. Die produkthaltigen Fraktionen werden eingeengt, in Isopropanol/Diisopropylether suspendiert, abgesaugt und mit Diisopropylether gewaschen. Ausbeute: 34.6 g (50%); Massenspektroskopie [M+H]⁺ = 298.

### d) 6-Benzyloxy-8-(2-chlor-acetyl)-4H-benzo[1,4]oxazin-3-on

13.8 g (46.0 mmol) 8-Acetyl-6-benzyloxy-4H-benzo[1,4]oxazin-3-on und 35.3 g (101.5 mmol) Benzyltrimethylammonium-dichloriodat werden in 250 mL Dichlorethan, 84 mL Eisessig und 14 mL Wasser 5 Stunden bei 65°C gerührt. Nach Abkühlung auf Raumtemperatur wird mit 5%iger Natriumhydrogensulfit-Lösung versetzt und 30 Minuten gerührt. Der ausgefallene Feststoff wird abgesaugt, mit Wasser und Diethylether gewaschen und getrocknet. Ausbeute: 13.2 g (86%); Massenspektroskopie [M+H]⁺ = 330/32.

### e) 6-Benzyloxy-8-((R)-2-chlor-1-hydroxy-ethyl)-4H-benzo[1,4]-oxazin-3-on

Die Durchführung erfolgt in Analogie zu einem in der Literatur beschriebenen Verfahren (Org. Lett. 2002, 4, 4373-4376).

Zu 13.15 g (39.6 mmol) 6-Benzyloxy-8-(2-chlor-acetyl)-4H-benzo[1,4]oxazin-3-on und 25.5 mg (0.04 mmol) Cp*RhCl[(S,S)-TsDPEN] (Cp* = Pentamethylcyclopentadienyl und TsDPEN = (1S,2S)-N-p-Toluensulfonyl-1,2-diphenylethylendiamin) in 40 mL Dimethylformamid werden bei -15°C 8 mL eines Gemisches aus Ameisensäure und Triethylamin (Molverhältnis = 5:2) getropft. Man lässt 5 Stunden bei dieser Temperatur Rühren, gibt dann 25 mg Katalysator nach und rührt über Nacht bei -15°C. Die Reaktionsmischung wird mit Eiswasser versetzt und filtriert. Der Filterrückstand wird in Dichlormethan gelöst, mit Natriumsulfat getrocknet und vom Lösungsmittel befreit. Der Rückstand wird chromatographiert (Dichlormethan/Methanol-Gradient) und das Produkt in Diethylether/Diisopropylether umkristallisiert. Ausbeute: 10.08 g (76%); R_{f}-Wert = 0.28 (Dichlormethan:Methanol = 50:1 auf Kieselgel).

### f) 6-Benzyloxy-8-(R)-oxiranyl-4H-benzo[1,4]oxazin-3-on

10.06 g (30.1 mmol) 6-Benzyloxy-8-((R)-2-chlor-1-hydroxy-ethyl)-4H-benzo[1,4]-oxazin-3-on werden in 200 mL Dimethylformamid gelöst. Die Lösung wird bei 0°C mit 40 mL einer 2 molaren Natronlauge versetzt und bei dieser Temperatur 4 Stunden gerührt. Die Reaktionsmischung wird auf Eiswasser gegossen, 15 Minuten gerührt und dann filtriert. Der Feststoff wird mit Wasser gewaschen und getrocknet. Ausbeute: 8.60 g (96%); Massenspektroskopie [M+H]⁺ = 298.

### g) 6-Benyloxy-8-{(R)-1-hydroxy-2-[2-(4-methoxy-phenyl)-1,1-dimethyl-ethylamino]-ethyl}-4H-benzo[1,4,]oxazin-3-on

5.25 g (17.7 mmol) 6-Benzyloxy-8-(R)-oxiranyl-4H-benzo[1,4]oxazin-3-on und 6.30 g (35.1 mmol) 2-(4-Methoxy-phenyl)-1,1-dimethyl-ethylamin werden mit 21 mL Isopropanol versetzt und 30 Minuten bei 135°C unter Mikrowelleneinstrahlung in einem geschlossenen Reaktionsgefäß gerührt. Das Lösungsmittel wird abdestilliert und der Rückstand chromatographiert (Aluminiumoxid; Ethylacetat/Methanol-Gradient). Das so erhaltene Produkt wird durch Umkristallisation aus einem Diethylether/Diisopropylether-Gemisch weiter gereinigt. Ausbeute: 5.33 g (63%); Massenspektroskopie [M+H]⁺ = 477.

### h) 6-Hydroxy-8-{(R)-1-hydroxy-2-[2-4-methoxy-phenyl)-1,1-dimethyl-ethylamino]-ethyl}-4H-benzo[1,4,]oxazin-3-on-Hydrochlorid

Eine Suspension von 5.33 g (11.2 mmol) 6-Benyloxy-8-{(R)-1-hydroxy-2-[2-(4-methoxyphenyl)-1,1-dimethyl-ethylamino]-ethyl}-4H-benzo[1,4,]oxazin-3-on in 120 mL Methanol wird mit 0.8 g Palladium auf Kohle (10%ig) versetzt, auf 50°C erwärmt und bei 3 bar Wasserstoffdruck hydriert. Anschließend wird der Katalysator abgesaugt und das Filtrat eingeengt. Man löst den Rückstand in 20 mL Isopropanol und gibt 2.5 mL 5 molare Salzsäure in Isopropanol zu. Das Produkt wird mit 200 mL Diethylether ausgefällt, abgesaugt und getrocknet. Ausbeute: 4.50 g (95%, Hydrochlorid); Massenspektroskopie [M+H]⁺ = 387.

In analoger Art und Weise werden durch Umsetzung der Verbindung 6-Benzyloxy-8-(R)-oxiranyl-4H-benzo[1,4]oxazin-3-on (Beispiel 1, Stufe f) mit dem entsprechenden Amin die folgenden Verbindungen der Formel **1** erhalten.

### Beispiel 2: 8-{(R)-2-[2-(2,4-Difluor-phenyl)-1,1-dimethyl-ethylamino]-1-hydroxy-ethyl}-6-hydroxy-4H-benzo[1,4]oxazin-3-on-Hydrochlorid

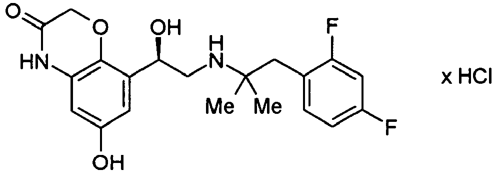

Massenspektroskopie [M+H]⁺ = 393.

### Beispiel 3: 8-{(R)-2-[2-(3,5-Difluor-phenyl)-1,1-dimethyl-ethylamino]-1-hydroxy-ethyl}-6-hydroxy-4H-benzo[1,4]oxazin-3-on-Hydrochlorid

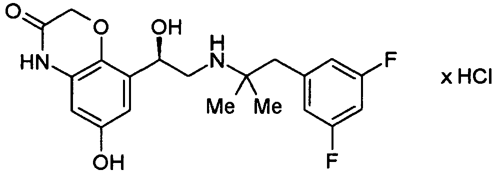

Massenspektroskopie [M+H]⁺ = 393.

### Beispiel 4: 8-{(R)-2-[2-(4-Ethoxy-phenyl)-1,1-dimethyl-ethylamino]-1-hydroxy-ethyl}-6-hydroxy-4H-benzo[1,4]oxazin-3-on-hydrochlorid

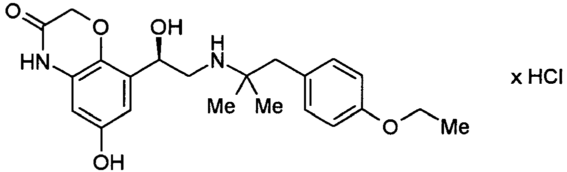

Massenspektroskopie [M+H]⁺ = 401.

### Beispiel 5: 8-{(R)-2-[2-(4-Fluor-phenyl)-1,1-dimethyl-ethylamino]-1-hydroxy-ethyl}-6-hydroxy-4H-benzo[1,4]oxazin-3-on- hydrochlorid

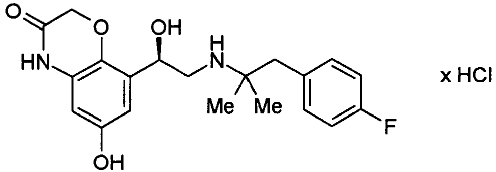

Massenspektroskopie [M+H]⁺ = 375.

Sofern die Verbindungen der Formel **1** gemäß der vorstehend exemplarisch angegebenen synthetischen Vorgehensweise gegebenenfalls nicht zu einheitlichen Kristallmodifikationen führen, kann es dienlich sein, die erhaltenen Salze der Formel **1** in geeigneten Lösemitteln umzukristallisieren. Darüber hinaus sind aus den vorstehend genannten Beispielen andere Salze durch Anwendung an und für sich im Stand der Technik bekannter Methoden zugänglich.

Im nachstehenden Teil werden examplarische Vorgehensweisen zur Darstellung einheitlicher und für die Darstellung von inhaltiv applizierbaren Darreichungsformen besonders geeigneter Salze der Verbindungen der Formel **1** ausgeführt.

### Beispiel 6: 6-Hydroxy-8-{(R)-1-hydroxy-2-[2-(4-methoxy-phenyl)-1,1-dimethyl-ethylamino]-ethyl}-4H-benzo[1,4,]oxazin-3-on maleinat

250 mg (0.65 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on werden mit soviel Ethanol versetzt, dass der Feststoff vollständig in Lösung geht. Anschließend werden 75 mg (0.65 mmol) Maleinsäure und eine Kristallisationshilfe zugegeben. Die Mischung wird mit Eis gekühlt und der ausgefallene Feststoff wird abfiltriert und mit Ethanol und Diethylether gewaschen. In dem Salz liegen die Säure und das Ethanolamin im Verhältnis 1:1 1 vor. Ausbeute: 254 mg (78%); Massenspektroskopie [M+H]⁺ = 387; Schmelzpunkt = 215°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 21,68 Å; 8,62 Å; 5,92 Å; 5,01 Å; 4,59 Å; 4,36 Å; 3,64 Å und 3,52 Å.

### Beispiel 7: 6-Hydroxy-8-{(R)-1-hydroxy-2-[2-(4-methoxy-phenyl)-1,1-dimethyl-ethylamino]-ethyl}-4H-benzo[1,4,]oxazin-3-on salicylat

250 mg (0.65 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on werden in wenig Ethanol gelöst und mit 90 mg (0.65 mmol) Salicylsäure versetzt. Nach Zugabe einer Kristallisationshilfe lässt man die Mischung über Nacht stehen, wobei ein Feststoff ausfällt. Es wird Diethylether zugesetzt und nach 30 Minuten filtriert. Der so erhaltene weiße Feststoff wird mit Diethylether gewaschen und getrocknet. Ausbeute: 295 mg (87%); Massenspektroskopie [M+H]⁺ = 387; Schmelzpunkt = 215°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 9,06 Å; 8,36 Å; 8,02 Å; 6,84 Å; 6,73 Å; 4,48 Å; 4,35 Å und 4,27 Å.

### Beispiel 8: 6-Hydroxy-8-{(R)-1-hydroxy-2-[2-(4-methoxy-phenyl)-1,1-dimethyl-ethylamino]-ethyl}-4H-benzo[1,4,]oxazin-3-on Succinat

500 mg (1.2 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid werden mit Ethylacetat versetzt und mit wässriger Kaliumcarbonat-Lösung ausgeschüttelt, die organische Phase wird mit Natriumsulfat getrocknet und vom Lösungsmittel befreit. Der Rückstand wird in wenig Ethanol gelöst und mit 140 mg (1.2 mmol) Bernsteinsäure versetzt. Nach 2 Stunden wird der ausgefallene Feststoff abgesaugt und mit kalten Ethanol und Diethylether gewaschen. In dem Salz liegen das Ethanolamin und die Säure im Verhältnis 1 zu 0.5 vor. Ausbeute: 468 mg (85%); Massenspektroskopie [M+H]⁺ = 387; Schmelzpunkt = 115°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 14,35 Å; 8,49 Å; 7,37 Å; 7,25 Å; 5,47 Å; 4,78 Å; 4,14 Å und 3,59 Å.

### Beispiel 9: 6-Hydroxy-8-{(R)-1-hydroxy-2-[2-(4-methoxy-phenyl)-1,1-dimethyl-ethylamino]-ethyl}-4H-benzo[1,4,]oxazin-3-on-fumarat

300 mg (0.71 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid werden mit Ethylacetat versetzt und mit wässriger Kaliumcarbonat-Lösung ausgeschüttelt. Die organische Phase wird mit Natriumsulfat getrocknet und vom Lösungsmittel befreit. Der Rückstand wird in Ethanol unter Zugabe von wenigen Tropfen Wasser gelöst. Man setzt 82 mg (0.71 mmol) Fumarsäure und Impfkristalle zu und lässt die Mischung über Nacht stehen. Der weiße Feststoff wird abgesaugt, mit Diethylether und Ethanol gewaschen und getrocknet. In dem Salz liegen das Ethanolamin und die Säure im Verhältnis 1 zu 0.5 vor. Ausbeute: 208 mg (63%); Massenspektroskopie [M+H]⁺ = 387; Schmelzpunkt = 130°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 14,23 Å; 5,44 Å; 4,76 Å; 4,57 Å; 4,26 Å; 4,12 Å; 3,57 Å und 3,48 Å.

### Beispiel 10: 6-Hydroxy-8-{1-hydroxy-2-[2-(4-methoxy-phenyl)-1,1-dimethyl-ethylamino]-ethyl}-4H-benzo[1,4,]oxazin-3-on (freie Base)

In Analogie zu den vorangegangenen Versuchen werden 500 mg (1.2 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid zunächst mit Ethylacetat versetzt. Die organische Phase wird mit wässriger Kaliumcarbonat-Lösung ausgeschüttelt, mit Natriumsulfat getrocknet und vom Lösungsmittel befreit. Die so erhaltene freie Base wird in Acetonitril unter Zugabe von wenigen Tropfen Wasser gelöst. Der ausfallende Feststoff wird abgesaugt, gewaschen und getrocknet. Ausbeute: 168 mg (37%); Massenspektroskopie [M+H]⁺ = 387; Schmelzpunkt = 128°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 14,96 Å; 9,63 Å; 7,05 Å; 5,57 Å; 5,28 Å; 5,05 Å; 4,63 Å und 3,73 Å.

### Beispiel 11: 6-Hydroxy-8-{(R)-1-hydroxy-2-[2-(4-methoxy-phenyl)-1,1-dimethyl-ethylamino]-ethyl}-4H-benzo[1,4,]oxazin-3-on-hydrochlorid

300 mg (0.71 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid werden durch Erwärmen in 4 mL Isopropanol gelöst. Die Lösung wird auf Raumtemperatur abgekühlt und dann für 15 Minuten in ein Eisbad gestellt. Der ausgefallene Feststoff wird abgesaugt und getrocknet. Ausbeute: 180 mg (60%); Massenspektroskopie [M+H]⁺ = 387; Schmelzpunkt = 211°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 5,92 Å; 5,81 Å; 5,51 Å; 5,10 Å; 4,65 Å; 4,50 Å; 4,15 Å und 4,00 Å.

Unter Anwendung der in den Beispielen 6 bis 11 dargestellten Vorgehensweise sind in analoger Weise die folgenden Verbindungen erhältlich:

### Beispiel 12: 8-{(R)-2-[2-(2,4-Difluor-phenyl)-1,1-dimethyl-ethylamino]-1-hydroxyethyl}-6-hydroxy-4H-benzo[1,4]oxazin-3-on-maleinat;

### Beispiel 13: 8-{(R)-2-[2-(2,4-Difluor-phenyl)-1,1-dimethyl-ethylamino]-1-hydroxyethyl}-6-hydroxy-4H-benzo[1,4]oxazin-3-on-salicylat;

### Beispiel 14: 8-{(R)-2-[2-(2,4-Difluor-phenyl)-1,1-dimethyl-ethylamino]-1-hydroxyethyl}-6-hydroxy-4H-benzo[1,4]oxazin-3-on-succinat;

### Beispiel 15: 8-{(R)-2-[2-(2,4-Difluor-phenyl)-1,1-dimethyl-ethylamino]-1-hydroxyethyl}-6-hydroxy-4H-benzo[1,4]oxazin-3-on-fumarat;

### Beispiel 16: 8-{(R)-2-[2-(3,5-Difluor-phenyl)-1,1-dimethyl-ethylamino]-1-hydroxyethyl}-6-hydroxy-4H-benzo[1,4]oxazin-3-on-maleinat;

### Beispiel 17: 8-{(R)-2-[2-(3,5-Difluor-phenyl)-1,1-dimethyl-ethylamino]-1-hydroxyethyl}-6-hydroxy-4H-benzo[1,4]oxazin-3-on-salicylat;

### Beispiel 18: 8-{(R)-2-[2-(3,5-Difluor-phenyl)-1,1-dimethyl-ethylamino]-1-hydroxyethyl}-6-hydroxy-4H-benzo[1,4]oxazin-3-on-succinat;

### Beispiel 19: 8-{(R)-2-[2-(3,5-Difluor-phenyl)-1,1-dimethyl-ethylamino]-1-hydroxyethyl}-6-hydroxy-4H-benzo[1,4]oxazin-3-on-fumarat;

### Beispiel 20: 8-{(R)-2-[2-(4-Ethoxy-phenyl)-1,1-dimethyl-ethylamino]-1-hydroxy-ethyl}-6-hydroxy-4H-benzo[1,4]oxazin-3-on-maleinat;

### Beispiel 21: 8-{(R)-2-[2-(4-Ethoxy-phenyl)-1,1-dimethyl-ethylamino]-1-hydroxy-ethyl}-6-hydroxy-4H-benzo[1,4]oxazin-3-on-salicylat;

### Beispiel 22: 8-{(R)-2-[2-(4-Ethoxy-phenyl)-1,1-dimethyl-ethylamino]-1-hydroxy-ethyl}-6-hydroxy-4H-benzo[1,4]oxazin-3-on-succinat;

### Beispiel 23: 8-{(R)-2-[2-(4-Ethoxy-phenyl)-1,1-dimethyl-ethylamino]-1-hydroxy-ethyl}-6-hydroxy-4H-benzo[1,4]oxazin-3-on-fumarat;

### Beispiel 24: 8-{(R)-2-[2-(4-Fluor-phenyl)-1,1-dimethyl-ethylamino]-1-hydroxy-ethyl}-6-hydroxy-4H-benzo[1,4]oxazin-3-on-maleinat;

### Beispiel 25: 8-{(R)-2-[2-(4-Fluor-phenyl)-1,1-dimethyl-ethylamino]-1-hydroxy-ethyl}-6-hydroxy-4H-benzo[1,4]oxazin-3-on-salicylat;

### Beispiel 26: 8-{(R)-2-[2-(4-Fluor-phenyl)-1,1-dimethyl-ethylamino]-1-hydroxy-ethyl}-6-hydroxy-4H-benzo[1,4]oxazin-3-on-succinat;

### Beispiel 27: 8-{(R)-2-[2-(4-Fluor-phenyl)-1,1-dimethyl-ethylamino]-1-hydroxy-ethyl}-6-hydroxy-4H-benzo[1,4]oxazin-3-on-fumarat;

### Beispiel 28: 8-{(R)-2-[2-(2,4-Difluor-phenyl)-1,1-dimethyl-ethylamino]-1-hydroxyethyl}-6-hydroxy-4H-benzo[1,4]oxazin-3-on (freie Base);

### Beispiel 29: 8-{(R)-2-[2-(3,5-Difluor-phenyl)-1,1-dimethyl-ethylamino]-1-hydroxyethyl}-6-hydroxy-4H-benzo[1,4]oxazin-3-on (freie Base);

### Beispiel 30: 8-{(R)-2-[2-(4-Ethoxy-phenyl)-1,1-dimethyl-ethylamino]-1-hydroxy-ethyl}-6-hydroxy-4H-benzo[1,4]oxazin-3-on (freie Base) oder auch

### Beispiel 31: 8-{(R)-2-[2-(4-Fluor-phenyl)-1,1-dimethyl-ethylamino]-1-hydroxy-ethyl}-6-hydroxy-4H-benzo[1,4]oxazin-3-on (freie Base).

### Darstellung der erfindungsgemäßem Pulverformulierungen:

### I) Apparatives

Zur Herstellung der Inhalationspulver können beispielsweise die folgenden Maschinen und Geräte Verwendung finden:
Mischbehälter bzw. Pulvermischer: Turbulamischer 2 L, Typ 2C; Hersteller Willy A. Bachofen AG, CH-4500 Basel
Handsieb: 0,315 mm Maschenweite

Die Befüllung der leeren Inhaltionskapseln mittels wirkstoffhaltigem Inhaltionspulver kann händisch oder maschinell erfolgen. Es können nachstehende Geräte verwendet werden.

### Kapselfüllmaschine:

MG2, Typ G100, Hersteller: MG2 S.r.l, I-40065 Pian di Macina di Pianoro (BO), Italien

### Beispiel 1:

### Pulvermischung :

Zur Herstellung der Pulvermischung werden 97,0 g Hilfsstoff (Lactose Monohydrat 200 Mesh mit einer je nach Charge variierenden mittleren Teilchengrösse von 25 - 50 µm) und 3,0 g mikronisierte Verbindung der Formel **1** eingesetzt. In den daraus erhaltenen 100g Inhalationspulver beträgt der Wirkstoffanteil 3,0 %.

Über ein Handsieb mit einer Maschenweite von 0,315 mm werden in einen geeigneten Mischbehälter Hilfsstoff vorgelegt. Anschließend werden abwechselnd 3g mikronisierte Verbindung der Formel **1** und 7g Hilfsstoff schichtweise eingesiebt. Die Zugabe des Hilfsstoffs und des Wirkstoffs erfolgt in 7 bzw. 6 Schichten (Vormischung I).

Die eingesiebten Bestandteile werden anschließend gemischt (Mischen: 30 UpM / 30 min). Über das gleiche Handsieb mit einer Maschenweite von 0,315 mm werden abwechselnd 10g Vormischung I und 90g Hilfsstoff schichtweise in einen geeigneten Mischbehälter eingesiebt. Die Zugabe des Hilfsstoff und der Vormischung I erfolgt 8 -10 Schichten (Endmischung). Die eingesiebten Bestandteile werden anschließend gemischt (Mischen: 30 UpM / 30 min).

Gemäß oder in Analogie zu der in Beispiel 1 beschriebenen Vorgehensweise können folgende Inhalationspulver erhalten werden:

### Beispiel 2:

| | |
|---|---|
| Wirkstoff = Beispiel **6** | 0,6g |
| Lactose Monohydrat: | 99,4g |
| Total: | 100,0g |

### Beispiel 3:

| | |
|---|---|
| Wirkstoff = Beispiel **6** | 3,0g |
| Lactose Monohydrat: | 97,0g |
| Total: | 100,0g |

### Beispiel 4:

| | |
|---|---|
| Wirkstoff = Beispiel **9** | 0,6g |
| Lactose Monohydrat: | 99,4g |
| Total: | 100,0g |

### Beispiel 5:

| | |
|---|---|
| Wirkstoff = Beispiel **10** | 0,6g |
| Lactose Monohydrat: | 99,4g |
| Total: | 100,0g |

### Beispiel 6:

| | |
|---|---|
| Wirkstoff = Beispiel **11** | 3,0g |
| Lactose Monohydrat: | 97,0g |
| Total: | 100,0g |

### Beispiel 7:

| | |
|---|---|
| Wirkstoff = Beispiel **13** | 0,6g |
| Lactose Monohydrat: | 99,4g |
| Total: | 100,0g |

### Beispiel 8:

| | |
|---|---|
| Wirkstoff = Beispiel **11** | 0,6g |
| Lactose Monohydrat: | 99,4g |
| Total: | 100,0g |

### Beispiel 9:

| | |
|---|---|
| Wirkstoff = Beispiel **10** | 3,0g |
| Lactose Monohydrat: | 97,0g |
| Total: | 100,0g |

### Beispiel 10:

| | |
|---|---|
| Wirkstoff = Beispiel **13** | 0,6g |
| Lactose Monohydrat: | 99,4g |
| Total: | 100,0g |

### Beispiel 11:

| | |
|---|---|
| Wirkstoff = Beispiel **14** | 0,6g |
| Lactose Monohydrat: | 99,4g |
| Total: | 100,0g |

### Beispiel 12:

| | |
|---|---|
| Wirkstoff = Beispiel **14** | 3,0g |
| Lactose Monohydrat: | 97,0g |
| Total: | 100,0g |

### Beispiel 13:

| | |
|---|---|
| Wirkstoff = Beispiel **15** | 0,6g |
| Lactose Monohydrat: | 99,4g |
| Total: | 100,0g |

### Beispiel 14:

| | |
|---|---|
| Wirkstoff = Beispiel **8** | 0,6g |
| Lactose Monohydrat: | 99,4g |
| Total: | 100,0g |

### Beispiel 15:

| | |
|---|---|
| Wirkstoff = Beispiel **8** | 3,0g |
| Lactose Monohydrat: | 97,0g |
| Total: | 100,0g |

### Beispiel 16:

| | |
|---|---|
| Wirkstoff = Beispiel **7** | 0,6g |
| Lactose Monohydrat: | 99,4g |
| Total: | 100,0g |

### Beispiel 17:

| | |
|---|---|
| Wirkstoff = Beispiel **6** | 0,6g |
| Mannitol: | 99,4g |
| Total: | 100,0g |

### Beispiel 18:

| | |
|---|---|
| Wirkstoff = Beispiel **6** | 3,0g |
| Mannitol: | 97,0g |
| Total: | 100,0g |

### Beispiel 19:

| | |
|---|---|
| Wirkstoff = Beispiel **9** | 0,6g |
| Mannitol | 99,4g |
| Total: | 100,0g |

### Beispiel 20:

| | |
|---|---|
| Wirkstoff= Beispiel **10** | 0,6g |
| Mannitol: | 99,4g |
| Total: | 100,0g |

### Beispiel 21:

| | |
|---|---|
| Wirkstoff = Beispiel **11** | 3,0g |
| Mannitol: | 97,0g |
| Total: | 100,0g |

### Beispiel 22:

| | |
|---|---|
| Wirkstoff = Beispiel **13** | 0,6g |
| Mannitol: | 99,4g |
| Total: | 100,0g |

### Beispiel 23:

| | |
|---|---|
| Wirkstoff = Beispiel **11** | 0,6g |
| Mannitol: | 99,4g |
| Total: | 100,0g |

### Beispiel 24:

| | |
|---|---|
| Wirkstoff = Beispiel **10** | 3,0g |
| Mannitol: | 97,0g |
| Total: | 100,0g |

### Beispiel 25:

| | |
|---|---|
| Wirkstoff = Beispiel **13** | 0,6g |
| Mannitol: | 99,4g |
| Total: | 100,0g |

### Beispiel 26:

| | |
|---|---|
| Wirkstoff= Beispiel **14** | 0,6g |
| Mannitol: | 99,4g |
| Total: | 100,0g |

### Bespiel 27:

| | |
|---|---|
| Wirkstoff = Beispiel **14** | 3,0g |
| Mannitol: | 97,0g |
| Total: | 100,0g |

### Beispiel 28:

| | |
|---|---|
| Wirkstoff = Beispiel **15** | 0,6g |
| Mannitol: | 99,4g |
| Total: | 100,0g |

### Beispiel 29:

| | |
|---|---|
| Wirkstoff = Beispiel **8** | 0,6g |
| Mannitol: | 99,4g |
| Total: | 100,0g |

### Beispiel 30:

| | |
|---|---|
| Wirkstoff = Beispiel **8** | 3,0g |
| Mannitol: | 97,0g |
| Total: | 100,0g |

### Beispiel 31:

| | |
|---|---|
| Wirkstoff = Beispiel **7** | 0,6g |
| Mannitol: | 99,4g |
| Total: | 100,0g |

## Patentansprüche

1. Inhalationspulver, enthaltend ein oder mehrere enantiomerenreine Verbindungen der allgemeinen Formel **1** 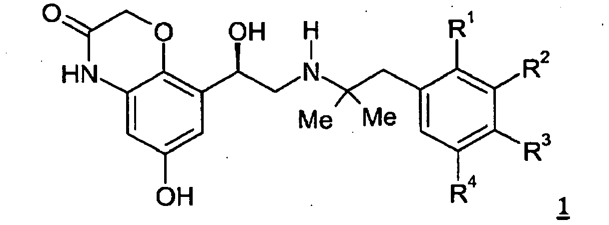 worin
R¹ Wasserstoff, C₁-C₄-Alkyl, C₁-C₄-Alkoxy oder Halogen;
R² Wasserstoff, C₁-C₄-Alkyl, C₁-C₄-Alkoxy oder Halogen;
R³ Wasserstoff, C₁-C₄-Alkyl, C₁-C₄-Alkoxy, Halogen, OH, -O-C₁-C₄-Alkylen-COOH oder -O-C₁-C₄-Alkylen-COO-C₁-C₄-alkyl;
R⁴ Wasserstoff, C₁-C₄-Alkyl, C₁-C₄-Alkoxy oder Halogen,
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen, **dadurch gekennzeichnet, dass** die Verbindungen der allgemeinen Formel **1** die Parameter aufweisen, charakteristische Partikelgröße X₅₀ von 0,1 µm bis 10µm und Q_{(5.8)} von größer 60%.

2. Inhalationspulver nach Anspruch 1, die ein oder mehrere enantiomerenreine Verbindungen der allgemeinen Formel **1** enthalten, worin
R¹ Wasserstoff oder Halogen;
R² Wasserstoff oder Halogen;
R³ Wasserstoff, C₁-C₄-Alkoxy oder Halogen;
R⁴ Wasserstoff oder Halogen;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen.

3. Inhalationspulver nach Anspruch 1 oder 2, die ein oder mehrere enantiomerenreine Verbindungen der allgemeinen Formel **1** enthalten, worin
R¹ Wasserstoff, Fluor oder Chlor, bevorzugt Wasserstoff oder Fluor;
R² Wasserstoff, Fluor oder Chlor, bevorzugt Wasserstoff oder Fluor;
R³ Wasserstoff, Methoxy, Ethoxy, Fluor oder Chlor, bevorzugt Wasserstoff, Methoxy, Ethoxy oder Fluor;
R⁴ Wasserstoff, Fluor oder Chlor, bevorzugt Wasserstoff oder Fluor;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen.

4. Inhalationspulver nach Anspruch 1, 2 oder 3, die ein oder mehrere enantiomerenreine Verbindungen der allgemeinen Formel **1** enthalten, worin
R¹ Wasserstoff oder Fluor;
R² Wasserstoff;
R³ Methoxy, Ethoxy oder Fluor;
R⁴ Wasserstoff;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen.

5. Inhalationspulver nach Anspruch 1, 2 oder 3, die ein oder mehrere enantiomerenreine Verbindungen der allgemeinen Formel **1** enthalten, worin
R¹ Wasserstoff;
R² Wasserstoff, Fluor oder Chlor, bevorzugt Wasserstoff oder Fluor;
R³ Wasserstoff;
R⁴ Wasserstoff, Fluor oder Chlor, bevorzugt Wasserstoff oder Fluor;
bedeuten, gegebenenfalls in Form ihrer pharmazeutisch verträglichen Säureadditionsalze, Hydrate oder Solvate, gegebenenfalls im Gemisch mit einem oder mehreren physiologisch unbedenklichen Hilfsstoffen.

6. Inhalationspulver nach einem der Ansprüche 1 bis 5, die ein oder mehrere enantiomerenreine Verbindungen der allgemeinen Formel **1** in Form ihrer freien Basen enthalten

7. Inhalationspulver nach einem der Ansprüche 1 bis 5, die ein oder mehrere enantiomerenreine Verbindungen der allgemeinen Formel **1** in Form ihrer pharmazeutisch verträglichen Säureadditionssalze enthalten.

8. Inhalationspulver nach einem der Ansprüche 1 bis 5 oder 7, die ein oder mehrere enantiomerenreine Verbindungen der allgemeinen Formel **1** in Form ihrer Saureadditionssalze der allgemeinen Formel **1-HX** enthalten, 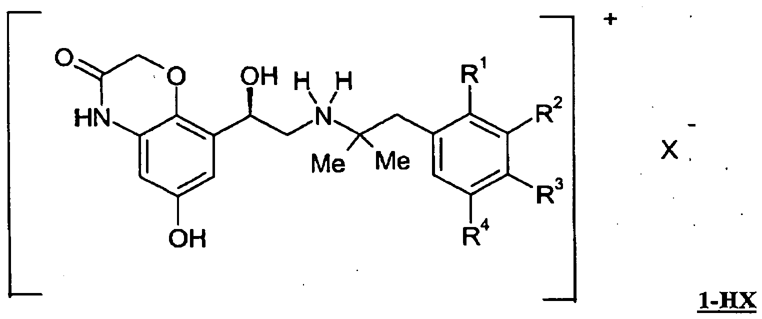 worin
X⁻ ein einfach negativ geladenes Anion, bevorzugt ein einfach negativ geladenes Anion ausgewählt aus der Gruppe bestehend aus Chlorid, Bromid, Iodid, Sulfat, Phosphat, Methansulfonat, Nitrat, Maleinat, Acetat, Benzoat, Citrat, Salicylat, Trifluoracetat, Fumarat, Tartrat, Oxalat, Succinat, Benzoat und p-Toluolsulfonat,
bedeutet und die Reste R¹, R², R³ und R⁴ die in den Ansprüchen 1 bis 5 genannten Bedeutungen aufweisen können, gegebenenfalls in Form Ihrer Tautomere, Mischungen der Tautomere, Hydrate oder Solvate, sowie gegebenenfalls im Gemisch mit einem oder mehreren physiologisch unbedenklichen Hilfsstoffen.

9. Inhalationspulver nach einem der Ansprüche 1 bis 8, **dadurch gekennzeichnet, dass** sie lediglich eine oder mehrere Verbindungen der allgemeinen Formel **1** enthalten.

10. Inhalationspulver nach einem der Ansprüche 1 bis 8, **dadurch gekennzeichnet, dass** sie neben einer oder mehrerer Verbindungen der allgemeinen Formel **1** wenigstens einen physiologisch verträglichen Hilfsstoff enthalten.

11. Inhalationspulver nach Anspruch 10, **dadurch gekennzeichnet, dass** der physiologisch verträgliche Hilfsstoff ausgewählt ist aus der Gruppe bestehend aus Monosaccharide, Disaccharide, Oligo- und Polysaccharide, Polylaktid-/glycolid, Polyalkohole, Aminosäuren, Chitosan, Alkali- und Erdalkalisalzen der Stearinsäure, Salze und Mischungen dieser Hilfsstoffe miteinander.

12. Inhalationspulver nach Anspruch 11, **dadurch gekennzeichnet, dass** der physiologisch verträgliche Hilfsstoff ausgewählt ist aus der Gruppe bestehend aus Glucose, Fructose, Arabinose, Lactose, Saccharose, Maltose, Trehalose, Maltodextrin, Stärke, Cellulose, Resomer, Sorbit, Mannit, Xylit, Argininhydrochlorid, Mg-Stearat, Natriumchlorid, Calciumcarbonat und Mischungen dieser Hilfsstoffe miteinander.

13. Inhalationspulver nach einem der Ansprüche 1 bis 12, **dadurch gekennzeichnet, dass** das Verhältnis von Verbindung der Formel **1** zu physiologisch verträgliche Hilfsstoff in einem Bereich von 5:100 bis 1:100000, bevorzugt 3:1000 bis 1:10000 liegt.

14. Inhalationspulver nach einem der Ansprüche 1 bis 12, **dadurch gekennzeichnet, dass** der physiologisch verträgliche Hilfsstoff eine mittlere Teilchengröße von etwa 17 - 120 µm, bevorzugt von etwa 17- 90 µm aufweist.

15. Verwendung eine Inhalationspulvers nach einem der Ansprüche 1 bis 14 zur Herstellung eines Arzneimittels zur Behandlung von Atemwegserkrankungen.

16. Pharmazeutischen Formulierung, **gekennzeichnet durch** einen Gehalt an einem Inhalationspulver gemäß einem der Ansprüche 1 bis 14.

## Claims

1. Inhalable powders containing one or more enantiomerically pure compounds of general formula **1** 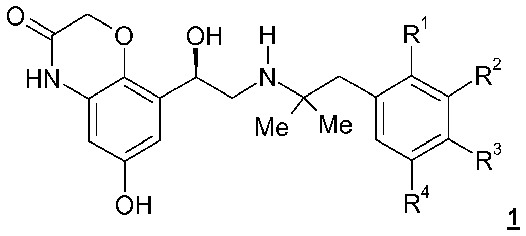 wherein
R¹ denotes hydrogen, C₁-C₄-alkyl, C₁-C₄-alkoxy or halogen;
R² denotes hydrogen, C₁-C₄-alkyl, C₁-C₄-alkoxy or halogen;
R³ denotes hydrogen, C₁-C₄-alkyl, C₁-C₄-alkoxy, halogen, OH, -O-C₁-C₄-alkylene-COOH or -O-C₁-C₄-alkylene-COO-C₁-C₄-alkyl;
R⁴ denotes hydrogen, C₁-C₄-alkyl, C₁-C₄-alkoxy or halogen,
optionally in the form of the pharmaceutically acceptable acid addition salts, hydrates or solvates thereof, optionally in admixture with one or more physiologically acceptable excipients, **characterised in that** the compounds of general formula **1** have the parameters of a characteristic particle size X₅₀ of from 0.1 µm to 10µm and Q_{(5.8)} of more than 60%.

2. Inhalable powders according to claim 1 which contain one or more enantiomerically pure compounds of general formula **1**, wherein
R¹ denotes hydrogen or halogen;
R² denotes hydrogen or halogen;
R³ denotes hydrogen, C₁-C₄-alkoxy or halogen;
R⁴ denotes hydrogen or halogen;
optionally in the form of the pharmaceutically acceptable acid addition salts, hydrates or solvates thereof, optionally in admixture with one or more physiologically acceptable excipients.

3. Inhalable powders according to claim 1 or 2 which contain one or more enantiomerically pure compounds of general formula **1**, wherein
R¹ denotes hydrogen, fluorine or chlorine, preferably hydrogen or fluorine;
R² denotes hydrogen, fluorine or chlorine, preferably hydrogen or fluorine;
R³ denotes hydrogen, methoxy, ethoxy, fluorine or chlorine, preferably hydrogen, methoxy, ethoxy or fluorine;
R⁴ denotes hydrogen, fluorine or chlorine, preferably hydrogen or fluorine;
optionally in the form of the pharmaceutically acceptable acid addition salts, hydrates or solvates thereof, optionally in admixture with one or more physiologically acceptable excipients.

4. Inhalable powders according to claim 1, 2 or 3 which contain one or more enantiomerically pure compounds of general formula **1**, wherein
R¹ denotes hydrogen or fluorine;
R² denotes hydrogen;
R³ denotes methoxy, ethoxy or fluorine;
R⁴ denotes hydrogen;
optionally in the form of the pharmaceutically acceptable acid addition salts, hydrates or solvates thereof, optionally in admixture with one or more physiologically acceptable excipients.

5. Inhalable powders according to claim 1, 2 or 3 which contain one or more enantiomerically pure compounds of general formula **1**, wherein
R¹ denotes hydrogen;
R² denotes hydrogen, fluorine or chlorine, preferably hydrogen or fluorine;
R³ denotes hydrogen;
R⁴ denotes hydrogen, fluorine or chlorine, preferably hydrogen or fluorine;
optionally in the form of the pharmaceutically acceptable acid addition salts, hydrates or solvates thereof, optionally in admixture with one or more physiologically acceptable excipients.

6. Inhalable powders according to one of claims 1 to 5 which contain one or more enantiomerically pure compounds of general formula **1** in the form of their free bases.

7. Inhalable powders according to one of claims 1 to 5 which contain one or more enantiomerically pure compounds of general formula **1** in the form of their pharmaceutically acceptable acid addition salts.

8. Inhalable powders according to one of claims 1 to 5 or 7 which contain one or more enantiomerically pure compounds of general formula **1** in the form of their acid addition salts of general formula **1-HX** 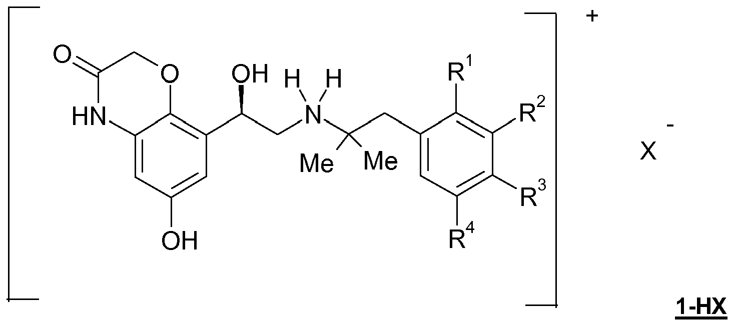 wherein
X⁻ denotes an anion with a single negative charge, preferably an anion with a single negative charge selected from the group consisting of chloride, bromide, iodide, sulphate, phosphate, methanesulphonate, nitrate, maleate, acetate, benzoate, citrate, salicylate, trifluoroacetate, fumarate, tartrate, oxalate, succinate, benzoate and p-toluenesulphonate,
and the groups R¹, R², R³ and R⁴ may have the meanings given in claims 1 to 5, optionally in the form of the tautomers, mixtures of the tautomers, hydrates or solvates thereof, as well as optionally in admixture with one or more physiologically acceptable excipients.

9. Inhalable powders according to one of claims 1 to 8, **characterised in that** they contain only one or more compounds of general formula **1**.

10. Inhalable powders according to one of claims 1 to 8, **characterised in that** they contain in addition to one or more compounds of general formula **1** at least one physiologically acceptable excipient.

11. Inhalable powders according to claim 10, **characterised in that** the physiologically acceptable excipient is selected from the group consisting of monosaccharides, disaccharides, oligo- and polysaccharides, polylactide/glycolide, polyalcohols, amino acids, chitosan, alkali metal and alkaline earth metal salts of stearic acid, salts and mixtures of these excipients with one another.

12. Inhalable powders according to claim 11, **characterised in that** the physiologically acceptable excipient is selected from the group comprising glucose, fructose, arabinose, lactose, saccharose, maltose, trehalose, maltodextrin, starch, cellulose, resomer, sorbitol, mannitol, xylitol, arginine hydrochloride, Mg-stearate, sodium chloride, calcium carbonate and mixtures of these excipients with one another.

13. Inhalable powders according to one of claims 1 to 12, **characterised in that** the ratio of compound of formula **1** to physiologically acceptable excipient is within the range from 5:100 to 1:100000, preferably 3:1000 to 1:10000.

14. Inhalable powders according to one of claims 1 to 12, **characterised in that** the physiologically acceptable excipient has an average particle size of about 17 -120 µm, preferably about 17- 90 µm.

15. Use of an inhalable powder according to one of claims 1 to 14 for preparing a medicament for the treatment of respiratory complaints.

16. Pharmaceutical formulation, **characterised in that** it contains an inhalable powder according to one of claims 1 to 14.

## Revendications

1. Poudres à inhaler, contenant un ou plusieurs composés énantiomères de formule générale 1 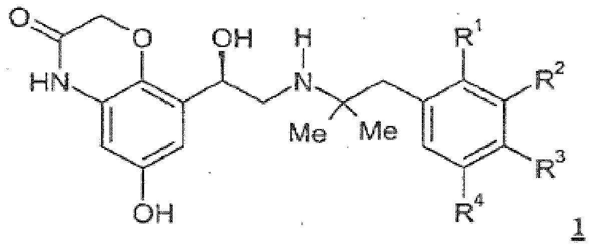 dans laquelle
R¹ désigne un atome d'hydrogène, un groupe alkyle en C₁ à C₄, alcoxy en C₁ à C₄ ou un atome d'halogène ;
R² désigne un atome d'hydrogène, un groupe alkyle en C₁ à C₄, alcoxy en C₁ à C₄ ou un atome d'halogène ;
R³ désigne un atome d'hydrogène, un groupe alkyle en C₁ à C₄, alcoxy en C₁ à C₄, un atome d'halogène, OH, -O-alkylène en C₁ à C₄-COOH ou -O-alkylène en C₁ à C₄-COO-alkyle en C₁ à C₄ ;
R⁴ désigne un atome d'hydrogène, un groupe alkyle en C₁ à C₄, alcoxy en C₁ à C₄ ou un atome d'halogène,
éventuellement sous la forme de leurs sels d'addition acide, d'hydrates ou de solvates pharmaceutiquement acceptables, éventuellement en mélange avec un ou plusieurs adjuvants physiologiquement inoffensifs, **caractérisées en ce que** les composés de formule générale 1 présentent les paramètres de taille caractéristique de particule X₅₀ de 0,1 µm à 10 µm et Q_{(5,8)} supérieur à 60 %.

2. Poudres à inhaler selon la revendication 1, qui contiennent un ou plusieurs composés énantiomères de formule générale 1, dans laquelle
R¹ désigne un atome d'hydrogène ou d'halogène ;
R² désigne un atome d'hydrogène ou d'halogène ;
R³ désigne un atome d'hydrogène, un groupe alcoxy en
C₁ à C₄ ou un atome d'halogène ;
R⁴ désigne un atome d'hydrogène ou d'halogène ;
éventuellement sous la forme de leurs sels d'addition acide, d'hydrates ou de solvates pharmaceutiquement acceptables, éventuellement en mélange avec un ou plusieurs adjuvants physiologiquement inoffensifs.

3. Poudres à inhaler selon la revendication 1 ou 2, qui contiennent un ou plusieurs composés énantiomères de formule générale 1, dans laquelle
R¹ désigne un atome d'hydrogène, de fluor ou de chlore, de préférence, d'hydrogène ou de fluor ;
R² désigne un atome d'hydrogène, de fluor ou de chlore, de préférence, d'hydrogène ou de fluor ;
R³ désigne un atome d'hydrogène, un groupe méthoxy, éthoxy, un atome de fluor ou de chlore, de préférence, d'hydrogène, un groupe méthoxy, éthoxy ou un atome de fluor ;
R⁴ désigne un atome d'hydrogène, de fluor ou de chlore, de préférence, d'hydrogène ou de fluor ;
éventuellement sous la forme de leurs sels d'addition acide, d'hydrates ou de solvates pharmaceutiquement acceptables, éventuellement en mélange avec un ou plusieurs adjuvants physiologiquement inoffensifs.

4. Poudres à inhaler selon la revendication 1, 2 ou 3, qui contiennent un ou plusieurs composés énantiomères de formule générale 1, dans laquelle
R¹ désigne un atome d'hydrogène ou de fluor ;
R² désigne un atome d'hydrogène ;
R³ désigne un groupe méthoxy, éthoxy ou un atome de fluor ;
R⁴ désigne un atome d'hydrogène ;
éventuellement sous la forme de leurs sels d'addition acide, d'hydrates ou de solvates pharmaceutiquement acceptables, éventuellement en mélange avec un ou plusieurs adjuvants physiologiquement inoffensifs.

5. Poudres à inhaler selon la revendication 1, 2 ou 3, qui contiennent un ou plusieurs composés énantiomères de formule générale 1, dans laquelle
R¹ désigne un atome d'hydrogène ;
R² désigne un atome d'hydrogène, de fluor ou de chlore, de préférence d'hydrogène ou de fluor ;
R³ désigne un atome d'hydrogène ;
R⁴ désigne un atome d'hydrogène, de fluor ou de chlore, de préférence, d'hydrogène ou de fluor ;
éventuellement sous la forme de leurs sels d'addition acide, d'hydrates ou de solvates pharmaceutiquement acceptables, éventuellement en mélange avec un ou plusieurs adjuvants physiologiquement inoffensifs.

6. Poudres à inhaler selon l'une des revendications 1 à 5, qui contiennent un ou plusieurs composés énantiomères de formule générale 1, sous la forme de leurs bases libres.

7. Poudres à inhaler selon l'une des revendications 1 à 5, qui contiennent un ou plusieurs composés énantiomères de formule générale 1, sous la forme de leurs sels d'addition acides pharmaceutiquement acceptables.

8. Poudres à inhaler selon l'une des revendications 1 à 5 ou 7, qui contiennent un ou plusieurs composés énantiomères de formule générale 1, sous la forme de leurs sels d'addition acide de formule générale 1-HX, 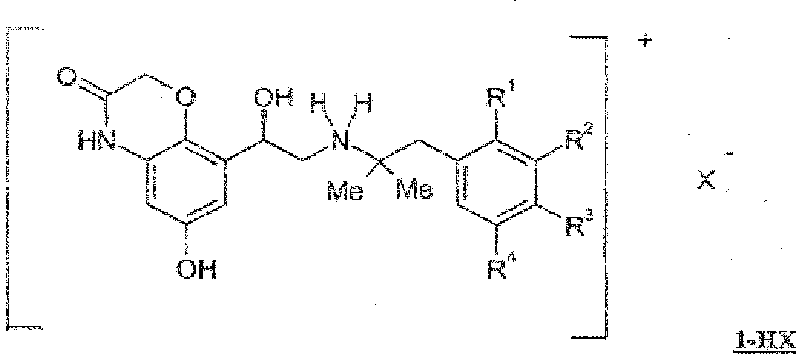 dans laquelle
X⁻ désigne un anion à charge négative, de préférence un anion à charge négative simple choisi dans le groupe comprenant le chlorure, le bromure, l'iodure, le sulfate, le phosphate, le méthanesulfonate, le nitrate, le maléinate, l'acétate, le benzoate, le citrate, le salicylate, le trifluoro-acétate, le fumarate, le tartrate, l'oxalate, le succinate, le benzoate et le p-toluène-sulfonate, et les radicaux R¹, R², R³ et R⁴ peuvent présenter les significations nommées dans les revendications 1 à 5, éventuellement sous la forme de leurs tautomères, de mélanges de tautomères, d'hydrates ou de solvates, et éventuellement en mélange avec un ou plusieurs adjuvants physiologiquement inoffensifs.

9. Poudres à inhaler selon l'une des revendications 1 à 8, **caractérisées en ce qu'**elles contiennent uniquement un ou plusieurs composés de formule générale 1.

10. Poudres à inhaler selon l'une des revendications 1 à 8, **caractérisées en ce qu'**elles contiennent, outre un ou plusieurs composés de formule générale 1, au moins un adjuvant physiologiquement acceptable.

11. Poudres à inhaler selon la revendication 10, **caractérisées en ce que** l'adjuvant physiologiquement acceptable est choisi dans le groupe comprenant les monosaccharides, les disaccharides, les oligo- et polysaccharides, le polylactide/ glycolide, les polyalcools, les acides aminés, le chitosan, les sels de métal alcalin et alcalino-terreux d'acide stéarique, les sels et mélanges de ces adjuvants les uns avec les autres.

12. Poudres à inhaler selon la revendication 11, **caractérisées en ce que** l'adjuvant physiologiquement acceptable est du groupe comprenant le glucose, le fructose, l'arabinose, le lactose, le saccharose, le maltose, le tréhalose, la maltodextrine, l'amidon, la cellulose, le résomère, le sorbitol, le mannitol, le xylitol, le chlorhydrate d'arginine, le stéarate de Mg, le chlorure de sodium, le carbonate de calcium et des mélanges de ces adjuvants les uns avec les autres.

13. Poudres à inhaler selon l'une des revendications 1 à 12, **caractérisées en ce que** le rapport du composé de formule 1 à l'adjuvant physiologiquement acceptable se situe dans une plage de 5:100 à 1:100000, de préférence, de 3:1000 à 1:10000.

14. Poudres à inhaler selon l'une des revendications 1 à 12, **caractérisées en ce que** l'adjuvant physiologiquement acceptable présente une taille de particule moyenne d'environ 17 à 120 µm, de préférence d'environ 17 à 90 µm.

15. Utilisation d'une poudre à inhaler selon l'une des revendications 1 à 14, pour la production d'un médicament pour le traitement de maladies des voies respiratoires.

16. Formulation pharmaceutique, **caractérisée par** une teneur en une poudre à inhaler selon l'une des revendications 1 à 14.
